# EUROPÄISCHE PATENTSCHRIFT

(11) **EP 0 890 344 B1**
(45) Veröffentlichungstag und Bekanntmachung des Hinweises auf die Patenterteilung: **06.10.2004**
(21) Anmeldenummer: 98112677.4
(22) Anmeldetag: 08.07.1998
(51) Int. Cl.: A61C 1/00, A61B 17/16

(54) **Ärztliche Behandlungsvorrichtung**
Medical treatment device
Dispositif de traitement médical

(30) Priorität: 08.07.1997 DE 19729178
(43) Veröffentlichungstag der Anmeldung: 13.01.1999
(73) Patentinhaber: Kaltenbach & Voigt GmbH & Co. KG, 88400 Biberach/Riss (DE)
(72) Erfinder: Eibofner, Eugen, 88400 Biberach (DE); Strohmaier, Ernst, 88427 Bad Schussenried (DE)
(74) Vertreter: Schmidt-Evers, Jürgen, Dipl.-Ing.

(56) Entgegenhaltungen:
- DE-A- 19 628 854
- US-A- 4 359 906
- US-A- 4 723 911
- US-A- 4 792 735

## Beschreibung

Die vorliegende Erfindung betrifft eine ärztliche Behandlungsvorrichtung. Insbesondere betrifft die vorliegende Erfindung eine zahnärztliche Behandlungsvorrichtung, welche im Bereich der Mikrochirurgie, HNO-Chirurgie, plastischen Chirurgie oder Kieferchirurgie sowie der Implantologie eingesetzt werden kann, wobei ein Antriebsmotor von einem Steuergerät der Behandlungsvorrichtung gesteuert wird und mit dem Antriebsmotor ein Behandlungsinstrument bzw. ein mit dem Behandlungsinstrument gekoppeltes Behandlungswerkzeug betrieben wird.

Bekannterweise besteht eine derartige ärztliche Behandlungsvorrichtung aus einem elektronischen Steuergerät, an das mit Hilfe einer biegsamen bzw. flexiblen Versorgungsleitung ein Behandlungsinstrument in Form eines Handstücks, z.B. in Form eines zahnärztlichen Handstücks oder eines zahnärztlichen Winkelstücks, anschließbar ist, welches an seinem vorderen Ende ein vorzugsweise austauschbares Behandlungswerkzeug, wie z.B. einen Bohrer, trägt. Der Antrieb des Behandlungsinstruments bzw. des Behandlungswerkzeugs erfolgt mit Hilfe eines Antriebsmotors, der an die Versorgungsleitung angeschlossen und mit dem Behandlungsinstrument koppelbar ist.

Das Steuergerät steuert über den Antriebsmotor den Betriebs des Behandlungsinstruments bzw. Behandlungswerkzeugs. Dabei kann das Steuergerät sowohl die Drehzahl als auch das Drehmoment des Antriebsmotors steuern, um ein bestimmtes Behandlungsinstrument bzw. Behandlungswerkzeug mit den jeweils geeigneten Betriebsparametern zu betreiben. Bei bestimmten Behandlungsinstrumenten bzw. Behandlungswerkzeugen, wie z.B. Bohrern, ist die Zufuhr eines Kühlmittels zur Behandlungsstelle erforderlich. Zu diesem Zweck ist eine Kühlmittelpumpe vorgesehen, die vorzugsweise am Steuergerät angebaut oder in dieses integriert sein kann. Über eine Kühlmittelleitung wird der Behandlungsstelle eine Kühlflüssigkeit, wie z.B. eine NaCl-Lösung, zugeführt, wobei die Kühlmittelleitung zusätzlich zu der Versorgungsleitung vorgesehen oder zumindest teilweise in die Versorgungsleitung bzw. das Handstück integriert sein kann. Neben dem Betriebs des Antriebsmotors kann dabei das Steuergerät auch den Betrieb der Kühlmittelpumpe, d.h. die zugeführte Kühlmittelmenge, steuern.

Hinsichtlich des Betriebs eines bestimmten Behandlungsinstruments bzw. Behandlungswerkzeugs ist es bekannt, an dem Steuergerät das gewünschte Drehmoment bzw. die gewünschte Drehzahl für den Antriebsmotor einzustellen. Nach Auswahl bzw. Einstellung des gewünschten Drehmoments bzw. der gewünschten Drehzahl wird der Antriebsmotor und das damit gekoppelte Behandlungsinstrument und Behandlungswerkzeug gemäß den eingestellten Betriebsparametern betrieben. Es ist auch bekannt, an dem Steuergerät Programmtasten vorzusehen, die mit voreingestellten Betriebsparameterwerten belegt sind. So ist von der Firma W. & H. Dentalwerk Bürmoos GmbH unter der Bezeichnung Elcomed 100/200 ein zahnärztliches Chirurgiegerät bekannt, bei dem das Steuergerät drei Programmtasten aufweist. Jeder Programmtaste ist herstellerseitig ein bestimmtes Behandlungsprogramm zugeordnet, wobei jedes Behandlungsprogramm eine voreingestellte Drehzahl, ein voreingestelltes Drehmoment, ein voreingestelltes Übersetzungsverhältnis und eine voreingestellte Kühlmittelpumpenfördermenge umfaßt. Durch Betätigen einer dieser drei Programmtasten werden der Antriebsmotor sowie die Kühlmittelpumpe gemäß den entsprechenden voreingestellten Betriebsparameterwerten betrieben.

Dem zuvor beschriebenen Stand der Technik ist gemeinsam, daß stets vom Steuergerät ein bestimmtes Drehmoment bzw. eine bestimmte Drehzahl dem Antriebsmotor vorgegeben wird, so daß der Antriebsmotor mit dem entsprechend vorgegebenen Betriebsparameterwert betrieben werden kann. Dabei können jedoch Schwankungen zwischen dem vorgegebenen Drehmoment und dem von dem tatsächlich benutzten Behandlungsinstrument und dem Antriebsmotor tatsächlich ausgeübten Drehmoment auftreten. Insbesondere hängt das Drehmoment nicht allein vom Antriebsmotorstrom, sondern auch vom Zustand des verwendeten Behandlungsinstruments ab, welches in der Regel auf den Antriebsmotor aufgesteckt wird. Selbst wenn - beispielsweise mit Hilfe entsprechender Programmtasten - an dem Steuergerät für den verwendeten Behandlungsinstrumententyp ein scheinbar geeignetes Drehmoment ausgewählt wird, kann es zwischen dem ausgewählten Drehmoment und dem von dem Antriebsmotor und dem damit gekoppelten Behandlungsinstrument tatsächlich ausgeübten Drehmoment aufgrund des Zustands des Antriebsmotors und des Behandlungsinstruments zu deutlichen Abweichungen kommen, wobei das tatsächlich von dem Antriebsmotor und dem Behandlungsinstrument ausgeübte Drehmoment mehr als 5 % von dem vorgegebenen bzw. eingestellten Drehmoment abweichen kann. Insbesondere bei zahnärztlichen Anwendungen, wie z.B. der Implantologie, sind jedoch zum Teil sehr genaue Drehmomente erforderlich.

Aus der DE-A1-196 28 854 ist ein Steuergerät für den Antriebsmotor eines zahnärztlichen Handstücks bekannt, welches eine Einrichtung zum Erfassen des auf ein Bohr- oder Fräswerkzeug aufgebrachten Lastdrehmoments aufweist. Sobald die Einrichtung erkannt hat, daß das von dem Antriebsmotor ausgeübte Lastdrehmoment einen voreingestellten Referenzdrehmomentwert überschritten hat, wird der Antriebsmotor entweder gestoppt oder die Drehzahl des Antriebsmotors gesenkt. Alternativ ist vorgesehen, die Drehrichtung des Antriebsmotors zeitweilig umzukehren. Mit Hilfe dieses Steuergeräts kann verhindert werden, daß ein Bohr- oder Fräswerkzeug, wie z.B. eine relativ schlanke Feile zur Ausbildung eines Wurzelkanals, aufgrund eines übermäßig hohen Drehmoments bricht. Das von dem Antriebsmotor ausgeübte Lastdrehmoment wird mit Hilfe eines Lastdrehmoment-Erfassungswiderstandes gemessen, welcher in Serie mit dem Antriebsmotor geschaltet ist, wobei die an dem Lastdrehmoment-Erfassungswiderstand abfallende Spannung als Maß für das Lastdrehmoment des Antriebsmotors ausgewertet wird.

Die in der DE-A1-196 28 854 beschriebene Steuerschaltung dient jedoch ausschließlich zur Überwachung des Drehmomentsin Abhängigkeit vom Motorstrom. Mit der darin beschriebenen Steuerschaltung kann jedoch nicht vermieden werden, daß der Antriebsmotor sowie das damit gekoppelte Behandlungsinstrument bzw. Behandlungswerkzeug mit einem geringfügig abweichenden Drehmomentwert betrieben wird. Insbesondere ist nicht sichergestellt, daß stets der Antriebsmotor mit dem damit gekoppelten Behandlungsinstrument mit dem tatsächlich von dem Antriebsmotor und dem Behandlungsinstrument ausgeübten Drehmoment betrieben wird. Zudem wird nicht das tatsächlich von einem Behandlungsinstrument ausgeübte Drehmoment gemessen, sondern lediglich abhängig vom Motorstrom auf das Drehmoment geschlossen.

Aus der WO 95/20146 A1 ist es bekannt, mit Hilfe einer Kalibrierungsvorrichtung das von einem zahnärztlichen Handstück in Abhängigkeit von dem Motorstrom erzeugte Drehmoment zu erfassen. Die zuvor erhaltenen Informationen werden dann benutzt, um das während einer Behandlung aktuell vorliegende Drehmoment anzuzeigen, so dass der Benutzer des Handstücks feststellen kann, ob ein zahnärztliches Implantat in ausreichender Weise festgeschraubt wurde oder nicht. Aufgrund der zuvor durchgeführten Kalibrierung kann somit das Drehmoment mit größerer Genauigkeit bestimmt werden, allerdings werden die erhaltenen Informationen lediglich dazu verwendet, dem Benutzer des Handstücks eine Rückmeldung über die Höhe des von dem Handstück erzeugten Drehmoments zu geben. Anhand dieser Informationen bleibt es dann dem Benutzer überlassen, ein gewünschtes Drehmoment einzustellen.

Der vorliegenden Erfindung liegt daher die Aufgabe zugrunde, eine ärztliche Behandlungsvorrichtung der zuvor beschriebenen Art derart auszugestalten, daß ein mit dem Antriebsmotor gekoppeltes Behandlungsinstrument bzw. Behandlungswerkzeug mit größtmöglicher Genauigkeit betrieben werden kann. Insbesondere soll das Behandlungsinstrument mit größtmöglicher Genauigkeit hinsichtlich des Drehmoments betrieben werden können.

Diese Aufgabe wird gemäß der vorliegenden Erfindung durch eine ärztliche Behandlungsvorrichtung nach Anspruch 1 gelöst.

Die Merkmale des Oberbegriffs des Anspruchs 1 Sind aus WO 95/20146 A1 bekannt.

Die Unteransprüche beschreiben allgemein vorteilhafte Ausgestaltungen und bevorzugte Ausführungsbeispiele der vorliegenden Erfindung.

Gemäß der vorliegenden Erfindung weist die ärztliche Behandlungsvorrichtung eine Drehmomentermittlungseinrichtung auf, welche vor Inbetriebnahme des Antriebsmotors sowie des damit gekoppelten Behandlungsinstruments das tatsächlich von dem Antriebsmotor und dem Behandlungsinstrument ausgeübte Drehmoment bzw. die entsprechende Drehmomentkennlinie erfaßt, wobei das Steuergerät den Antriebsmotor mit dem damit gekoppelten Behandlungsinstrument abhängig von den somit gewonnenen Drehmomentdaten ansteuert. Auf diese Weise kann eine Abweichung zwischen der dem Steuergerät ursprünglich vorgegebenen Drehmomentkennlinie und der tatsächlich von dem Antriebsmotor mit dem Behandlungsinstrument ausgeübten Drehmomentkennlinie korrigiert werden, so daß das Behandlungsinstrument mit der insbesondere für Anwendungen in der Implantologie erforderlichen Abweichungsgenauigkeit des Drehmoments < ± 3 % betrieben werden kann.

Die Drehmomentermittlungseinrichtung kann in das Steuergerät der ärztlichen Behandlungsvorrichtung integriert oder als externe Einheit ausgestaltet sein.

Die Ermittlung der Drehmomentdaten kann insbesondere dadurch erfolgen, daß die Drehmomentermittlungseinrichtung für vorgegebene Drehmomentwerte die dem Antriebsmotor entsprechend zugeführte Leistung ermittelt und mit den Drehmomentwerten korreliert.

Vor Inbetriebnahme des Antriebsmotors mit dem daran gekoppelten Behandlungsinstrument wird somit gemäß der vorliegenden Erfindung eine Drehmomentkalibrierung durchgeführt. Dieser Drehmomentkalibrierungsvorgang wird vorteilhafterweise dadurch erleichtert, daß die Drehmomentermittlungseinrichtung ein Steuermodul mit einer beispielsweise menügesteuerten Benutzerführung aufweist.

Die bevorzugt gemäß der vorliegenden Erfindung angewendete Drehmomentermittlungseinrichtung besitzt Erfassungsmittel, welche die Drehbewegung des Behandlungsinstruments erfassen und in eine Verstellkraft für ein Verstellmedium umsetzen. Des weiteren sind Umwandlungsmittel vorgesehen, welche den durch die Verstellkraft hervorgerufenen Verstellweg des Verstellmediums erfassen und davon abhängig ein entsprechendes elektrisches Signal liefern, welches in die entsprechenden Drehmomentdaten umgewandelt wird.

Die Messung des Verstellwegs des Verstellmediums wird insbesondere mit Hilfe einer Schalteranordnung durchgeführt, wobei die Schalteranordnung mehrere Schalter umfaßt, die abhängig von dem Verstellweg unterschiedlich betätigt werden.

Vor Inbetriebnahme eines mit dem Antriebsmotor gekoppelten Behandlungsinstruments wird dieses Behandlungsinstrument mit dem Antriebsmotor in die Drehmomentermittlungseinrichtung eingeführt, wobei insbesondere das Behandlungsinstrument mit einem Mitnehmer bzw. einer Aufnahmewelle der Drehmomentermittlungseinrichtung gekoppelt wird, so daß die Drehmomentermittlungseinrichtung das von dem Antriebsmotor mit dem Behandlungsinstrument in Kombination ausgeübte Drehmoment zuverlässig erfassen und eine entsprechende Drehmomentkalibrierung veranlassen kann.

Die Erfindung wird nachfolgend unter Bezugnahme auf die Zeichnung anhand bevorzugter Ausführungsbeispiele näher erläutert.
- Fig. 1: zeigt ein Ausführungsbeispiel einer erfindungsgemäßen ärztlichen Behandlungsvorrichtung,
- Fig. 2: zeigt eine vergrößerte Darstellung der Frontseite des in Fig. 1 dargestellten Steuergeräts,
- Fig. 3a: zeigt beispielhaft den Aufbau der in dem in Fig. 1 gezeigten Steuergerät gespeicherten Datenbank,
- Fig. 3b und 3c: zeigen die Kupplung eines Antriebsmotors mit einem dentalen Handstück bzw. Winkelstück sowie einem Behandlungswerkzeug,
- Fig. 4: zeigt ein schematisches Blockdiagramm zur Erläuterung der Erstellung eines benutzerspezifischen Behandlungs- bzw. Betriebsprogramms des in Fig. 2 gezeigten Steuergeräts,
- Fig. 5: zeigt beispielhaft herstellerseitig vorgegebene Betriebsprogramme des in Fig. 2 gezeigten Steuergeräts,
- Fig. 6: zeigt ein Ausführungsbeispiel einer einteiligen Ablage für Behandlungsinstrumente, Behandlungswerkzeuge sowie den Antriebsmotor, die bei der erfindungsgemäßen ärztlichen Behandlungsvorrichtung zum Einsatz kommen kann,
- Fig. 7: zeigt verschiedene Ansichten einer zweiteiligen Variante der in Fig. 6 gezeigten Ablage,
- Fig. 8: zeigt ein erstes Ausführungsbeispiel einer erfindungsgemäßen Einrichtung zur Drehmomentkalibrierung eines mit dem Antriebsmotor gekoppelten Behandlungsinstruments,
- Fig. 9: zeigt verschiedene Ansichten eines zweiten Ausführungsbeispiels einer erfindungsgemäßen Einrichtung zur Drehmomentkalibrierung,
- Fig. 10: zeigt die Kennlinie des Drehmoments gegenüber dem Antriebsmotorstrom zur Erläuterung der Drehmomentkalibrierung mit der in Fig. 8 bzw. 9 gezeigten Einrichtung, und
- Fig. 11: zeigt verschiedene Ansichten eines dritten Ausführungsbeispiels einer erfindungsgemäßen Einrichtung zur Drehmomentkalibrierung.

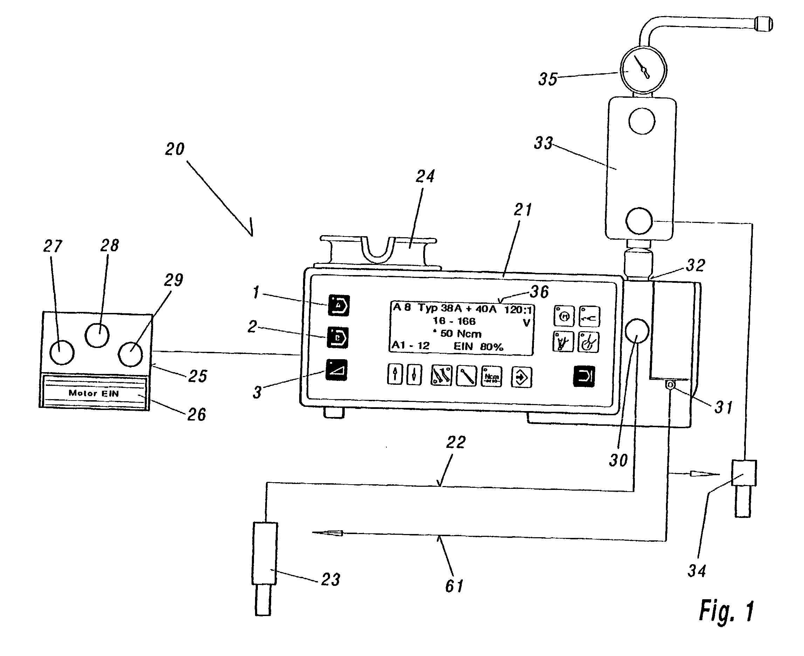

Fig. 1 zeigt ein bevorzugte Ausführungsbeispiel einer zahnärztlichen Behandlungsvorrichtung 20, wobei es sich insbesondere um eine zahnärztliche Behandlungsvorrichtung zum Einsatz in der Mikrochirurgie, HNO-Chirurgie, plastischen Chirurgie, Kieferchirurgie und Implantologie handelt.

Der zentrale Bestandteil der in Fig. 1 dargestellten ärztlichen Behandlungsvorrichtung ist ein Steuergerät 21, welches abhängig von vorgegebenen Betriebsparameterinformationen einen Antriebsmotor steuert, der mit einem Behandlungsinstrument sowie einem daran gekoppelten Behandlungswerkzeug koppelbar ist. Gemäß Fig. 1 ist der Antriebsmotor 23 über eine Versorgungsleitung 22 an einen entsprechenden Anschluß 30 angeschlossen. Die Versorgungsleitung 22 kann durch eine Schnellkupplung an dem Steuergerät, d.h. dem Anschluß 30, sowie durch eine Schraubkupplung an dem Antriebsmotor 23 befestigt sein. Als Antriebsmotor 23 wird vorzugsweise ein kollektorloser Motor mit Stellungsmelder verwendet, dessen Drehzahlbereich zwischen 200 und 40 000 Umdrehungen/min. liegt. Das Drehmoment dieses Antriebsmotors beträgt beispielsweise 5 Ncm bei einer Antriebsdrehzahl von 200/min. Auf den Antriebsmotor 23 kann - wie bereits erwähnt worden ist - ein Behandlungsinstrument, wie z.B. ein zahnärztliches Handstück oder ein Reduzierwinkelstück, aufgesteckt werden, welches das eigentliche Behandlungswerkzeug, wie z.B. einen Bohrer od.dgl. trägt. Abhängig von der Drehzahlübertragung des mit dem Antriebsmotor gekoppelten Behandlungsinstruments können somit unterschiedliche Drehzahlbereiche abgedeckt und unterschiedliche maximale Drehmomentwerte erzielt werden.

Bei einer chirurgischen Behandlung wird in der Regel sowohl der Antriebsmotor als auch das Behandlungsinstrument und das Behandlungswerkzeug mit von dem behandelten Körper stammenden Krankheitserregern kontaminiert. Aus diesem Grund ist es erforderlich, daß sowohl der Antriebsmotor 23 als auch die Versorgungsleitung 22 autoklavierbar und somit leicht sterilisierbar sind.

Für bestimmte Behandlungsarten, bei denen eine Wärmeentwicklung an der Behandlungsstelle auftritt, ist die Zufuhr eines Kühlmittels erforderlich. Aus diesem Grund weist die in Fig. 1 gezeigte ärztliche Behandlungsvorrichtung eine Kühlmittelpumpe auf, die über einen entsprechenden Anschluß 31 ein Kühlmittel, wie z.B. eine NaCl-Salzlösung, der Behandlungsstelle zuführt. Die an den Anschluß 31 angeschlossene Kühlmittelleitung 61 kann extern zusammen mit der Versorgungsleitung 22 dem Antriebsmotor 23 bzw. dem daran angekoppelten Behandlungsinstrument zugeführt werden. Alternativ kann jedoch die Kühlmittelleitung 61 auch zumindest teilweise in die Versorgungsleitung 22 bzw. das Antriebsmotor-Handstück 23 integriert sein. Wie nachfolgend noch näher beschrieben wird, steuert das Steuergerät 21 der ärztlichen Behandlungsvorrichtung 20 die Zufuhr des Kühlmittels über die Kühlmittelleitung 61 und regelt insbesondere die zugeführte Kühlmittelmenge.

Die in Fig. 1 gezeigte ärztliche Behandlungsvorrichtung 20 kann wahlweise auch mit zwei Anschlüssen 30 und zwei Antriebsmotoren 23 ausgestaltet sein, die insbesondere unterschiedliche Drehzahlbereiche oder Drehmomentwerte aufweisen, um somit unterschiedliche Anwendungsgebiet abdecken zu können. Die beiden Antriebsmotore können unabhängig voneinander betrieben werden. Vorzugsweise ist dem zweiten Antriebsmotor eine zweite Kühlmittelpumpe zugeordnet, die analog zu der zuvor beschriebenen Kühlmittelpumpe mit der entsprechenden Kühlmittelleitung 61 ausgestaltete sein kann.

Eine Besonderheit der in Fig. 1 gezeigten ärztlichen Behandlungsvorrichtung 20 ist die Verwendung einer Pneumatik-Antriebseinrichtung 33, die an einen Luftanschluß 32 des Steuergeräts angeschlossen ist. Diese Pneumatik-Antriebseinrichtung 33 ist zum Betreiben bestimmter Sonder-Behandlungsinstrumente vorgesehen, die zum einen Luftantrieb und zum anderen eine sterile Kühlflüssigkeit erfordern und an einen Multiflex-Anschluß 34 angeschlossen werden. Bei diesen Sonder-Behandlungsinstrumenten kann es sich um bestimmte Instrumente für die Paradontologie, Kronen- und Brückenabnahme, Endodontie und Wurzelspitzenresektion handeln. Der von der Pneumatik-Antriebseinrichtung 33 gelieferte Antriebsluftdruck kann mit Hilfe der Pneumatik-Antriebseinrichtung 33 sowie einem entsprechend vorgesehenen Luftdruckmesser 35 überwacht und beliebig reguliert und eingestellt werden. Des weiteren kann mit Hilfe des Steuergeräts 21 der Luftdruck sowie die zugeführte Kühlflüssigkeitsmenge der Pneumatik-Antriebseinrichtung 33 gesteuert werden.

Wie aus Fig. 1 ersichtlich ist, weist die Frontseite der Steuereinrichtung 21 eine integrierte Anzeigeneinheit 36 auf, welche - wie noch nachfolgend ausführlicher zu beschreiben ist - die augenblicklich ausgewählten bzw. geltenden Betriebsparameterinformationen anzeigt. Da bei Aktivierung der Pneumatik-Antriebseinrichtung 33 der Antrieb des daran angeschlossenen Sonder-Behandlungsinstruments 34 durch Luftdruck erfolgt, kann die Anzeigeneinheit 36 in diesem Fall die geltenden Luftdruckeinstellungen der Pneumatik-Antriebseinrichtung 33 zusammen mit der geltenden Kühlmitteleinstellung anzeigen. Ebenso ist denkbar, daß auf der Anzeigeneinheit 36 in diesem Fall ausschließlich die Kühlmitteldaten wiedergegeben werden.

Des weiteren ist gemäß Fig. 1 eine nachfolgend noch näher zu beschreibende Ablage 24 vorgesehen, die zur Ablage von Behandlungswerkzeugen, Behandlungsinstrumenten sowie des Antriebsmotors 23 vorgesehen ist. Bei der Ablage 24 handelt es sich um eine separate Multifunktionsablage, in die der Antriebsmotor 23 mit der dazugehörigen Versorgungsleitung 22 bzw. dem Versorgungsschlauch sowie benutzte Behandlungsinstrumente und Behandlungswerkzeuge abgelegt werden können. Wie nachfolgend noch näher beschrieben werden wird, kann die Ablage 24 sterilisiert werden und dient zugleich beim Sterilisieren als Verpackung für den darin abgelegten Antriebsmotor 23, das Behandlungsinstrument sowie den Versorgungsschlauch 22.

Es ist bereits bekannt, das Steuergerät 21 der ärztlichen Behandlungsvorrichtung 20 mit Hilfe eines Fußanlassers 25 zu betreiben, um eine manuelle Betätigung der Funktionstasten des Steuergeräts 21 während der Behandlung zu vermeiden (vgl. z.B. Druckschrift EP - A2 - 0525 539). Aus der JP-A-62 - 16106 ist ein Fußschalter zur drahtlosen Fernsteuerung eines zahnmedizinischen Stuhls bekannt. Dieses Prinzip ist bei der in Fig. 1 dargestellten ärztlichen Behandlungsvorrichtung angewendet, d.h. der Fußschalter 25 kommuniziert mit einer entsprechend in dem Steuergerät 21 vorgesehenen Empfangseinheit über eine kabellose Übertragungsstrecke, die z.B. durch eine Funk- oder Infrarot-Übertragungsstrecke gebildet sein kann. Der Fußschalter 25 weist demnach eine selbständige Energieversorgung in Form eines Akkumulators oder einer Batterie auf. Wie nachfolgend noch beschrieben wird, kann mit Hilfe der in Fig. 1 dargestellten ärztlichen Behandlungsvorrichtung zwischen hersteller- bzw. werkseitig vorgegebenen Betriebsprogrammen, die nicht von einem Benutzer veränderbar sind, und benutzerspezifisch erstellten und abgespeicherten Betriebsprogrammen ausgewählt werden.

Nach Auswahl der herstellerseitigen Betriebsprogrammebene bzw. der benutzerspezifischen Betriebsprogrammebene an dem Steuergerät 21 kann mit Hilfe des Schalters 28 der Benutzer über den Fußschalter 25 sämtliche Betriebsprogramme der ausgewählten Betriebsprogrammebene durchschalten und auf diese Weise ein gewünschtes Betriebsprogramm dieser Betriebsprogrammebene auswählen. Dies bedeutet, daß das Steuergerät nicht mit den (möglicherweise unsterilen) Fingern berührt und bedient werden muß, da die gesamte Steuerung über den Fußschalter 25 erfolgen kann. Alternativ kann der Fußschalter 25 auch einen Auswahlschalter zum Umschalten zwischen der herstellerseitigen, unveränderbaren Betriebsprogrammebene und der benutzerspezifischen, beliebig veränderbaren Betriebsprogrammebene aufweisen, so daß auch die Auswahl zwischen diesen beiden Betriebsprogrammebenen durch den Fußschalter 25 erfolgen kann. Über die Schalter 26, 27 und 29 kann der Benutzer manuell in das ausgewählte Betriebsprogramm eingreifen, wobei der Schalter 26 zur direkten Ansteuerung des Antriebsmotors 23 dient. Dieser Schalter 26 besitzt die Funktion eines Gaspedals und dient zum Ein- und Ausschalten des Antriebsmotors 23. Ebenso ist denkbar, daß über den Schalter 26 abhängig von dem Niederdrücken des Schalters 26 die Drehzahl des Antriebsmotors 23 eingestellt wird. Mit Hilfe des Schalters 27 kann die Kühlmittelpumpe 31 ein- und ausgeschaltet werden. Schließlich kann mit Hilfe des Schalters 29 der (standardmäßig) vorgegebene Rechtslauf des Antriebsmotors 23 auf Linkslauf und umgekehrt umgestellt werden. Vorteilhafterweise ist der Fußschalter 25 wasserdicht ausgestaltet, um stets die Funktionsfähigkeit des Fußschalters 25 zu gewährleisten.

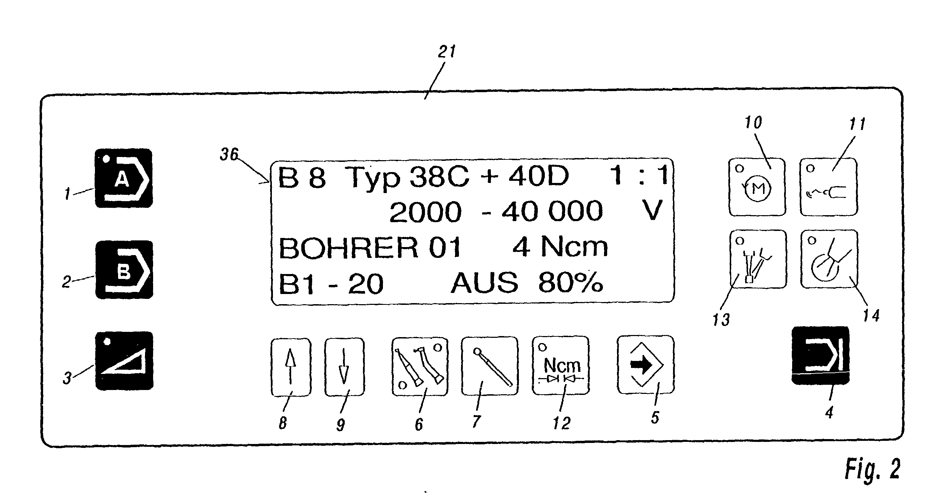

Nach der allgemeinen Beschreibung der Bestandteile der in Fig. 1 dargestellten ärztlichen Behandlungsvorrichtung soll nachfolgend unter Bezugnahme auf Fig. 2 - 5 die Funktionsweise dieser ärztlichen Behandlungsvorrichtung näher beschrieben werden.

Fig. 2 zeigt eine vergrößerte Darstellung der Frontseite des in Fig. 1 dargestellten Steuergerätes 21 der ärztlichen Behandlungsvorrichtung 20. In die Frontseite ist eine Anzeigeneinheit 36, z.B. eine Flüssigkristallanzeige integriert, die vorzugsweise vierzeilig die geltenden bzw. ausgewählten Betriebsparameterinformationen anzeigt, so daß eine optische Überwachung des Betriebs der ärztlichen Behandlungsvorrichtung möglich ist. Des weiteren weist die Frontseite des Steuergeräts 21 eine Vielzahl von Auswahltasten 1 - 14 auf, bei denen es sich insbesondere aus hygienischen Gründen um Folientasten handeln kann, die durch einen kurzen Tastendruck aktivierbar sind. Jeder dieser Tasten 1 - 14 ist eine bestimmte Funktion zugewiesen, die nachfolgend näher erläutert werden soll.

Die in Fig. 1 dargestellte ärztliche Behandlungsvorrichtung besitzt - wie bereits zuvor beschrieben worden ist - im wesentlichen zwei Programmebenen, wobei in der einen Programmebene aus herstellerseitigen Betriebsprogrammen zum Betreiben bestimmter Behandlungsinstrumente und Behandlungswerkzeuge ausgewählt werden kann. Diese werk- bzw. herstellerseitige Programmebene umfaßt ausschließlich Betriebsprogramme, die von einem Benutzer nicht verändert, d.h. nicht überschrieben werden können. Des weiteren ist eine benutzerspezifische Betriebsprogrammebene vorgesehen, die eine Vielzahl von benutzerspezifischen Betriebsprogrammen umfaßt, die zuvor von einem Benutzer erstellt und von diesem beliebig veränderbar sind. Die herstellerseitig vorgegebenen Betriebsprogramme sowie die benutzerspezifisch erstellten Betriebsprogramme werden in einem Speicher des Steuergeräts 21 abgelegt. Im Prinzip kann eine beliebige Anzahl von herstellerseitig vorgegebenen Betriebsprogrammen oder benutzerspezifischen Betriebsprogrammen gespeichert werden. Aus Gründen der Übersichtlichkeit und des Speicherplatzes wird jedoch vorteilhafterweise die maximal zulässige Anzahl von herstellerseitig vorgegebenen bzw. benutzerspezifisch erstellten Betriebsprogrammen beispielsweise auf jeweils 100 Betriebsprogramme begrenzt.

Nach Auswahl eines Behandlungs- bzw. Betriebsprogramms aus diesen beiden Programmebenen werden die entsprechenden Betriebsparameterinformationen des ausgewählten Betriebsprogramms auf der Anzeigeneinheit 36 dargestellt und der Antriebsmotor 23 kann gemäß diesen vorgegebenen Betriebsparameterinformationen durch das Steuergerät 21 ggfs. in Zusammenwirkung mit dem Fußschalter 25 angesteuert werden.

Neben diesen beiden Programmebenen ist bei der in Fig. 1 gezeigten ärztlichen Behandlungsvorrichtung auch ein Einstellbetrieb vorgesehen, in dem ein Benutzer nach Auswahl eines Betriebsprogramms manuell die dem Betriebsprogramm entsprechenden Betriebsparameterwerte verändern kann. Ebenso kann der Benutzer auch ohne vorherige Auswahl eines Betriebsprogramms die Betriebsparameter mit Hilfe der Funktionstasten des Steuergeräts 21 bzw. des Fußschalters 25 einstellen. In diesem Einstellmodus arbeitet der Benutzer vorwiegend mit Erfahrungs- bzw. Gefühlswerten.

Mit Hilfe der in Fig. 2 dargestellten Taste 1 kann ein Benutzer zwischen sämtlichen herstellerseitig vorgegebenen Betriebsprogrammen, die nicht von dem Benutzer verändert werden können, auswählen. Dabei kann vorgesehen sein, daß mit jeder Betätigung der Taste 1 das nächste herstellerseitig vorgegebene Betriebsprogramm ausgewählt und in der Anzeigeneinheit 36 angezeigt wird. In diesem Fall können sämtliche herstellerseitig vorgegebenen Betriebsprogramme lediglich durch Betätigung der Taste 1 durchblättert werden. Alternativ kann vorgesehen sein, daß durch Betätigung der Taste 1 zunächst in die herstellerseitig vorgegebene Programmebene gewechselt wird, in der dann anschließend mit Hilfe der Tasten 8 und 9 die einzelnen herstellerseitig vorgegebenen Betriebsprogramme durchblättert werden können. Auch in diesem Fall wird das jeweils geltende bzw. ausgewählte Betriebsprogramm bzw. die entsprechenden Betriebsparameterinformationen in der Anzeigeneinheit 36 dargestellt.

Mit Hilfe der Taste 2 kann analog zu der Taste 1 zwischen sämtlichen benutzerspezifisch erstellten Behandlungs- bzw. Betriebsprogrammen ausgewählt werden, wobei das Auswählen eines benutzerspezifisch erstellten Betriebsprogramms entweder allein durch Betätigen der Taste 2 oder in Kombination mit den Tasten 8 und 9 erfolgen kann. Auch die einem ausgewählten benutzerspezifisch erstellten Betriebsprogramm entsprechenden Betriebsparameterinformationen werden in der Anzeigeneinheit 36 dargestellt.

Wie bereits beschrieben worden ist, wird jedes Betriebsprogramm durch bestimmte Betriebsparameterinformationen definiert, die insbesondere den Typ des verwendeten Behandlungsinstruments, den Typ des verwendeten Behandlungswerkzeugs, technische Informationen zum Betrieb des Behandlungsinstruments bzw. des Behandlungswerkzeugs sowie Kühlmittelinformationen umfassen.

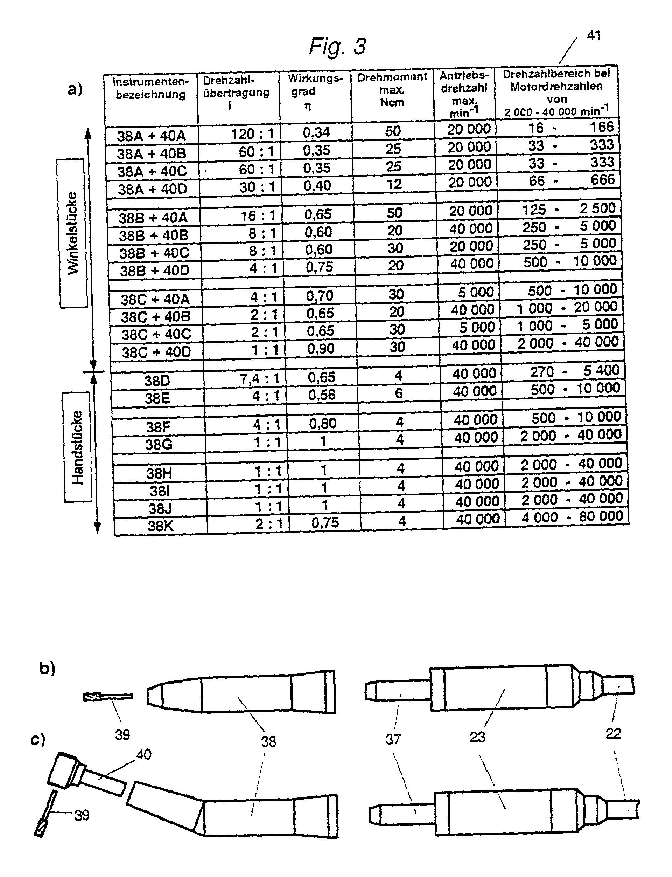

Wie in Fig. 3b und 3c gezeigt ist, wird im Falle einer zahnärztlichen Behandlungsvorrichtung auf die Antriebswelle 37 des über den Versorgungsschlauch 22 an das Steuergerät 21 angeschlossenen Antriebsmotors ein Behandlungsinstrument 38 aufgesetzt, wobei es sich gemäß Fig. 3b um ein Handstück und gemäß Fig. 3c um ein Winkelstück handelt. Ein Behandlungswerkzeug 39 wird mit dem aufgesetzten Behandlungsinstrument 38 gekoppelt, wobei im Falle eines in Fig. 3c dargestellten Winkelstücks 38 auf das Winkelstück 38 zudem ein Drehzahlübertragungskopf 40 mit ggfs. vorhandener Drehzahluntersetzung aufgesetzt werden kann, in den anschließend das Behandlungswerkzeug 39 eingeführt wird. Abhängig von dem Anwendungsgebiet ist eine Vielzahl von Behandlungsinstrumenten 38, Kopfteilen 40 und Behandlungswerkzeugen 39 bekannt. Während sich die Behandlungsinstrumente 38 bzw. Kopfteile 40 vorwiegend in der Drehzahlübertragung bzw. Drehzahluntersetzung unterscheiden, kann es sich bei den Behandlungswerkzeugen 39 beispielsweise um Körner, Bohrer, Fräser oder Gewindeschneider usw. handeln.

In dem Steuergerät 21 ist eine Datenbank vorhanden, die Informationen zu sämtlichen verfügbaren Behandlungsinstrumenten 38, Kopfteilen 40, Behandlungswerkzeugen 39 sowie den verfügbaren Kühlmittelmöglichkeiten enthält. Der die Behandlungsinstrumente 38 betreffende Datensatz 41 dieser Datenbank ist beispielhaft in Fig. 3a dargestellt. Daraus ist ersichtlich, daß der die Behandlungsinstrumente 38 betreffende Datensatz beispielsweise die Typ- bzw. Produktbezeichnung für verschiedene Winkelstücke 38A-C oder Handstücke 38D-K beinhaltet. Wird als Behandlungsinstrument 38 ein Winkelstück (Typ 38A-38C) eingesetzt, umfaßt die erste Spalte des in Fig. 3a gezeigten Datensatzes jeweils die Kombination eines Winkelstücks 38A-C mit einem entsprechenden Kopfteil 40A-D. Zu jedem Behandlungsinstrument bzw. zu jeder Kombination eines Winkelstücks mit einem Kopfteil umfaßt der in Fig. 3a gezeigte Datensatz zudem die sich daraus ergebenden technischen Eigenschaften, wie z.B. die daraus resultierende Drehzahlübertragung, der Wirkungsgrad, das maximale Drehmoment, die maximal zulässige Antriebsdrehzahl sowie der sich bei einer Antriebsdrehzahl des Antriebsmotors 23 von 2000 - 40 000 /min ergebende Drehzahlbereich. Es können theortetisch beliebig viele dieser Hand- und Winkelstückdatensätze in der Datenbank abgelegt werden.

Neben dem in Fig. 3a gezeigten behandlungsinstrumentenbezogenen Datensatz 41 umfaßt - wie bereits beschrieben worden ist - die Datenbank zudem einen behandlungswerkzeugbezogenen Datensatz sowie einen kühlmittelbezogenen Datensatz. Der behandlungswerkzeugbezogene Datensatz beschreibt beispielsweise für die einzelnen Typen von verfügbaren Behandlungswerkzeuge, wie z.B. Bohrer, die dazu gehörigen technischen Eigenschaften. Der kühlmittelbezogene Datensatz beschreibt die Kühlmittelmöglichkeiten, wie z.B. die einstellbaren Kühlmittelmengen oder Kühlmittelpumpenleistungen.

Diese drei zuvor beschriebenen Datensätze der in dem Steuergerät 21 abgelegten Datenbank können nunmehr in Einheiten als Betriebsprogramme zusammengefaßt werden. Diese Betriebsprogramme stellen somit jeweils eine Kombination eines bestimmten Behandlungsinstrumentes mit einem bestimmten Behandlungswerkzeug dar, wobei für diese Kombination bestimmte technische Betriebsparameter, wie z.B. das Drehmoment oder der Drehzahlbereich, sowie eine entsprechende Kühl mitteleinstellung vorgegeben sein kann. Somit können in der ersten und zweiten Programmebene für verschiedene Anwendungen unterschiedliche Betriebsprogramme vorgesehen sein.

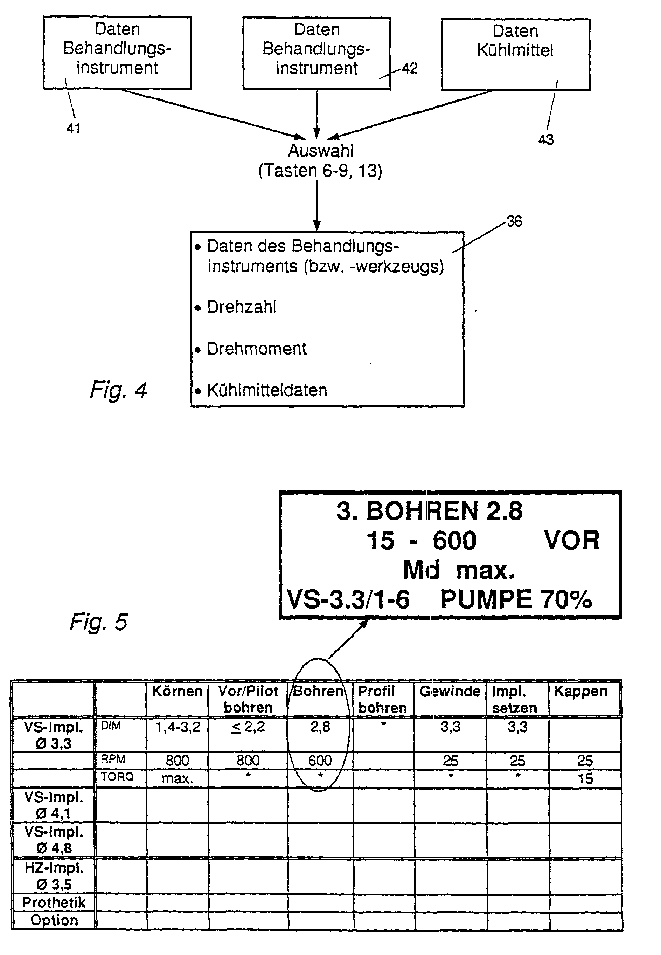

So zeigt Fig. 5 beispielsweise anhand der herstellerseitig vorgegebenen Programmebene, die von dem Benutzer nicht veränderbare Betriebsprogramme umfaßt, abhängig für in der ersten Spalte dargestellte Anwendungen unterschiedliche Betriebsprogrammfolgen. Dabei ist in der ersten Zeile in Fig. 5 beispielhaft das Setzen eines Implantats mit einem Durchmesser von 3,3 mm dargestellt. Diese Anwendung umfaßt gemäß Fig. 5 insgesamt sieben unterschiedliche entsprechend zugeordnete Betriebsprogramme, die mit "Körnen", "Vor/Pilotbohren", "Bohren", "Profilbohren", "Gewinde", "Implantatsetzen" und "Kappen" bezeichnet sind. Jedes dieser Betriebsprogramme umfaßt Informationen über den dabei verwendeten Bohrerdurchmesser ("DIM"), die benutzte Betriebsdrehzahl ("RPM") sowie das zulässige Drehmoment ("TORQ"). Der Benutzer kann somit abhängig von der gewählten Anwendung nacheinander die dieser Anwendung zugeordneten Betriebsprogramme durchlaufen bzw. diese auswählen, wobei nach Auswahl eines entsprechenden Betriebsprogramms die diesem Betriebsprogramm zugeordnete Betriebsparameterinformationen auf der Anzeigeneinheit 36 des Steuergerätes 21 dargestellt werden. Dies ist in Fig. 5 beispielhaft anhand des Betriebsprogramms "Bohren" angedeutet. In der ersten Zeile der Anzeigeneinheit 36 können Informationen über das verwendete Behandlungsinstrument bzw. Behandlungswerkzeug dargestellt sein. Gemäß Fig. 5 enthält die erste Zeile der Anzeigeneinheit 36 zudem mit der Bezeichnung "3." eine Information über die Sequenznummer des augenblicklich ausgewählten Betriebsprogramms der entsprechenden Anwendung. Die zweite Zeile der Anzeigeneinheit 36 umfaßt gemäß Fig. 5 Informationen über den dem Betriebsprogramm zugewiesenen Drehzahlbereich sowie den eingestellten Vorwärtsbetrieb ("VOR"). In der dritten Zeile wird das maximale Drehmoment angegeben, während die vierte Zeile gemäß Fig. 5 Informationen über die zugeführte Kühlmittelmenge (gemäß Fig. 5 70 %) und die Bezeichnung der dem ausgewählten Betriebsprogramm entsprechenden Anwendung enthalten kann. Selbstverständlich kann die Anzeigeneinheit 36 auch eine Zeilenanzahl größer als vier aufweisen und die dem ausgewählten Betriebsprogramm entsprechenden Betriebsparameterinformationen auch in anderer Form sowie anderer Reihenfolge darstellen.

Der Aufbau der Betriebsprogramme wurde zuvor anhand der unveränderbar herstellerseitig vorgegebenen Betriebsprogramme erläutert. Dieser Aufbau trifft jedoch genauso auf die benutzerspezifisch erstellten Betriebsprogramme zu. Wie bereits zuvor beschrieben worden ist, kann mit Hilfe der in Fig. 2 dargestellten Taste 2 sowie ggfs. in Kombination mit den Tasten 8 und 9 ein beliebiges benutzerspezifisch erstelltes Betriebsprogramm ausgewählt werden. Auch die in der zweiten Programmebene abgelegten benutzerspezifischen Betriebsprogramme umfassen demnach Informationen über den zu benutzenden Behandlungsinstrumententyp, ggfs. Kopfteiltyp, Behandlungswerkzeugtyp sowie die entsprechenden technischen Betriebsparameter, wie z.B. Drehzahlbereich oder Drehmoment. Bevor ein beliebiges benutzerspezifisch erstelltes Betriebsprogramm jedoch mit Hilfe der Taste 2 ausgewählt werden kann, muß zumindest ein derartiges benutzerspezifisches Betriebsprogramm erstellt worden sein. Dies soll nachfolgend unter Bezugnahme auf Fig. 4 näher erläutert werden.

Wie bereits zuvor beschrieben worden ist, kann mit Hilfe der beiden Tasten 1 und 2 ein beliebiges Betriebsprogramm der herstellerseitig vorgegebenen, unveränderbaren ersten Programmebene bzw. der benutzerspezifisch erstellten, beliebig veränderbaren zweiten Programmebene ausgewählt werden. Zudem ist ein Einstellmodus vorgesehen, der durch die in Fig. 2 dargestellte Taste 2 aufgerufen werden kann. In diesem Einstellmodus können sämtliche technische Daten durch den Benutzer frei eingestellt werden. Nach Betätigen der Taste 3 erscheint beispielsweise in der ersten Zeile der in Fig. 2 gezeigten Anzeigeeinheit 36 lediglich die Bezeichnung "frei", während in der zweiten bis vierten Zeile Informationen über die augenblicklich eingestellte Istdrehzahl des Antriebsmotors, den Drehmomentbereich des Antriebsmotors sowie den Betriebszustand der Kühlung angegeben werden. In diesem Einstellmodus arbeitet der Benutzer vorwiegend aufgrund seiner Erfahrungs- und Gefühlswerte, ohne daß ihm bestimmte Betriebsparameter eines gespeicherten Betriebsprogramms vorgegeben sind.

Des weiteren kann der Benutzer über die Taste 6 eine bestimmte Aufsatzkombination des Antriebsmotors einstellen. Dabei wird nach Betätigung der Taste 6 der beispielhaft in Fig. 3a dargestellte instrumentenbezogene Datensatz 41 der intern abgelegten Datenbank des Steuergeräts aufgerufen, wobei der Benutzer mit Hilfe der Tasten 8 und 9 durch die einzelnen Datensatzeinträge blättern kann. Der jeweils augenblicklich ausgewählte Datensatzeintrag wird dabei beispielsweise in der ersten Zeile der Anzeigeneinheit 36 dargestellt. Ebenso kann der Benutzer mit Hilfe der Taste 7 den behandlungswerkzeugbezogenen Datensatz aufrufen und wiederum mit Hilfe der Tasten 8 und 9 durch die einzelnen Datensatzeinträge blättern, wobei die jeweils gültige Auswahl ebenfalls auf der Anzeigeneinheit 36 dargestellt wird. Schließlich kann der Benutzer auch mit Hilfe der Taste 13 den vorgegebenen, in dem Steuergerät 21 abgelegten kühlmittelbezogenen Datensatz aufrufen und mit Hilfe der Tasten 8 und 9 die gewünschte Kühlmittelmenge bzw. Kühlmittelzufuhrleistung der Kühlmittelpumpe auswählen und einstellen, wobei die jeweils gültigen Kühlmitteldaten auch auf der Anzeigeneinheit 36 dargestellt werden. Des weiteren kann mit Hilfe der Taste 13 die Kühlmittelzufuhr einund ausgeschaltet werden.

Wie in Fig. 4 gezeigt ist, erfolgt somit mit Hilfe der Tasten 6 - 9 und 13 eine benutzerspezifische Auswahl der einzelnen Einträge des behandlungsinstrumentenbezogenen Datensatzes 41, des behandlungswerkzeugbezogenen Datensatzes 42 sowie des kühlmittelbezogenen Datensatzes 43, wobei die sich daraus ergebende Kombination der einzelnen Einstellungen auf der Anzeigeneinheit 36 dargestellt wird.

Durch Betätigen der Taste 5 kann schließlich der Benutzer diese Kombination als ein neues benutzerspezifisch erstelltes Behandlungs- bzw. Betriebsprogramm in der zu der Taste 2 gehörenden benutzerspezifischen Programmebene ablegen. Nach Betätigen der Taste 5 wird das neu erstellte benutzerspezifische Betriebsprogramm auf den nächsten freien Speicherplatz der benutzerspezifischen Programmebene abgelegt. Sollten sämtliche Speicherplätze dieser zweiten benutzerspezifischen Programmebene belegt sein, kann ein Warnton ausgegeben werden oder das neue Betriebsprogramm automatisch unter den ersten Speicherplatz der zweiten Programmebene abgelegt werden, wobei jedoch in diesem Fall das zuvor unter dem ersten Speicherplatz abgelegte Betriebsprogramm verlorengeht.

Neben der zuvor beschriebenen Vorgehensweise zur Erstellung eines benutzerspezifischen Betriebsprogramms kann der Benutzer auch ein gespeichertes Betriebsprogramm der den Auswahltasten 1 und 2 entsprechenden Programmebenen auswählen, mit Hilfe der Tasten 6 - 9 und 13 verändern und als neues benutzerspezifisches Betriebsprogramm abspeichern, wobei jedoch nur die unter der Taste 2 abgelegten benutzerspezifischen Betriebsprogramme überschrieben werden können.

Wie bereits zuvor erläutert worden ist, werden die einem ausgewählten Betriebsprogramm entsprechenden Betriebsparameterinformationen auf der Anzeigeneinheit 36 dargestellt. Dabei ist in Fig. 2 beispielhaft die Auswahl eines benutzerspezifischen Betriebsprogramms mit Hilfe der Taste 2 dargestellt. Wie in Fig. 2 gezeigt ist, gibt die erste Zeile der Anzeigeneinheit 36 nach Auswahl eines benutzerspezifischen Betriebsprogramms zunächst die Bezeichnung des Betriebsprogramms ("B 8") sowie den dem ausgewählten Betriebsprogramm entsprechenden Behandlungsinstrumententyp mit der sich daraus ergebenden Drehzahlübertragung wieder (vgl. auch Fig. 3a). Wie bereits anhand Fig. 3a beschrieben worden ist, handelt es sich in diesem Fall um die Kombination eines Winkelstücks vom Typ "38C" mit einem Kopfteil vom Typ "40D". In der zweiten Zeile ist der Drehzahlbereich angegeben, wobei als Betriebsart der Vorwärtsbetrieb ("V") festgelegt ist. In der dritten Zeile ist der Typ des zu verwendenden Behandlungswerkzeugs ("Bohrer 01") und das maximale Drehmoment dargestellt. In der vierten Zeile ist schließlich die Gesamtzahl der gespeicherten benutzerspezifischen Betriebsprogramme ("B 1 - 20") zusammen mit kühlmittelbezogenen Daten dargestellt. Gemäß der vierten Zeile beträgt die eingestellte Kühlmittelmenge 80 %, wobei jedoch die Kühlmittelzufuhr ausgeschaltet ist.

Nachfolgend sollen unter Bezugnahme auf Fig. 2 die restlichen Funktionstasten des Steuergerätes 21 beschrieben werden. Mit Hilfe der Taste 10 kann die Drehrichtung des Antriebsmotors von Vorwärtsbetrieb auf Rückwärtsbetrieb und umgekehrt umgeschaltet werden. Der Antriebsmotor weist zu der jeweils gewählten drehmomentbegrenzten Drehrichtung ein in die entgegengesetzte Drehrichtung erhöhtes Drehmoment auf. Durch die Taste 11 kann der in Fig. 1 dargestellte Pneumatik-Anschluß 32 aktiviert und deaktiviert werden. Durch Betätigung der Taste 12 kann eine nachfolgend noch näher zu beschreibende Drehmomentkalibrierungsprozedur aufgerufen werden, die zur Drehmomentkalibrierung des Antreibsmotors mit einem darauf aufgesetzten Behandlungsinstrument dient. Die Taste 13 dient - wie bereits beschrieben worden ist - zum Ein-/Ausschalten der Kühlmittelzufuhr, wobei in Kombination mit den Tasten 8 und 9 die Kühlmittelmenge eingestellt werden kann. Um Salzablagerungen infolge der NaCl-Salzlösung in der in Fig. 1 gezeigten Kühlmittelleitung 61 sowie der Sterilisation zu vermeiden, muß die Kühlmittelleitung 61 in regelmäßigen Abständen mit destilliertem Wasser durchgespült werden. Diese Spülung der Kühlmittelleitung kann durch Betätigung der Taste 4 ausgelöst werden, wobei die Spülung vorzugsweise nur gestartet wird, wenn tatsächlich zuvor Kühlmittel verwendet worden ist. Mit der Taste 4 können schließlich die einzelnen ausgewählten Betriebsprogramme, d.h. die Behandlung eines Patienten, beendet werden, wobei vorzugsweise nach Betätigung dieser Taste 4 auf der Anzeigeneinheit 36 Pflegehinweise sowie weitere hilfreiche Informationen angezeigt werden.

Zudem kann die Anzeigeeinheit 36 zur Anzeige von Fehlermeldungen dienen, die über während des Betriebs der ärztlichen Behandlungsvorrichtung 20 bzw. des Steuergeräts 21 auftretende Fehler informieren. So kann beispielsweise auf Fehler in der Elektronik des Steuergeräts 21, auf Fehler des Antriebsmotors 23 oder auf die Datenbank des Steuergeräts 21 betreffende Fehler, wenn z.B. sämtliche Speicherplätze der beiden den Tasten 1 und 2 zugeordneten Programmebenen belegt sind, hingewiesen werden. Ebenso ist denkbar, das Steuergerät 21 mit einer automatischen Behandlungstyp-Erkennungseinrichtung auszustatten, so daß eine Abweichung zwischen dem einem ausgewählten Betriebsprogramm entsprechenden Behandlungsinstrumententyp und dem tatsächlich auf den Antriebsmotor 23 aufgesteckten Behandlungsinstrumententyp erkannt und eine entsprechende Fehlermeldung ausgegeben werden kann. Derartige Behandlungsinstrumententyp-Erkennungseinrichtungen sind bereits bekannt.

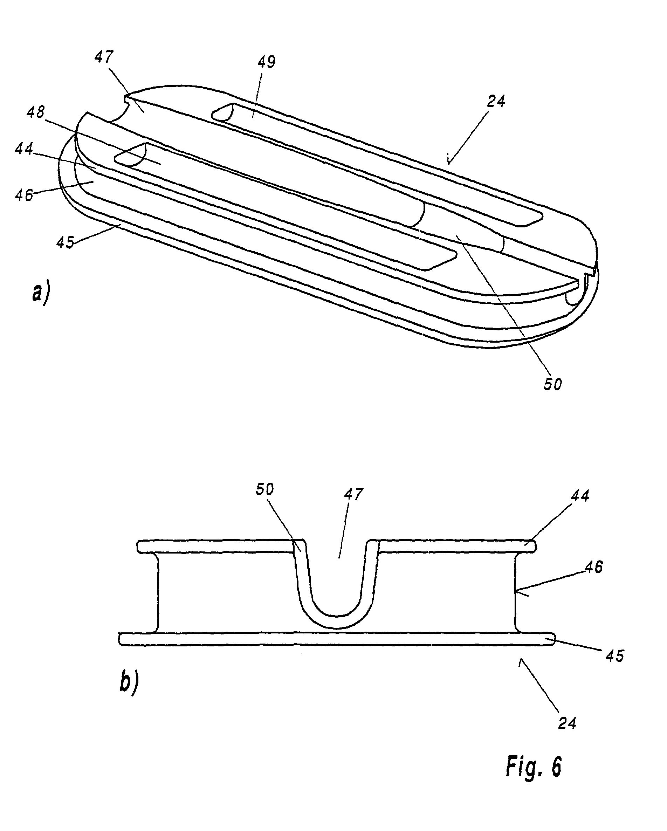

Fig. 6 zeigt ein erstes Ausführungsbeispiel der in Fig. 1 dargestellten Ablage 24, die zur Aufnahme des Antriebsmotors 23 sowie von Behandlungsinstrumenten und Behandlungswerkzeugen und dem Versorgungsschlauch 22 dient, wobei Fig. 6a eine perspektivische Ansicht dieses ersten Ausführungsbeispiels und Fig. 6b eine Frontalansicht dieses Ausführungsbeispiels darstellt.

Die in Fig. 6 dargestellte Ablage 24 besitzt eine sich in Längsrichtung der Ablage 24 erstreckende durchlaufende Vertiefung 47, die zur Aufnahme des in Fig. 1 dargestellten Antriebsmotors 23 dient. Die Vertiefung 47 ist im wesentlichen an die Außenform des Antriebsmotors 23 angepaßt und weist an ihrem einen Ende eine umfangsseitige Verengung auf, so daß der in der Vertiefung 47 abgelegte Motor in der Richtung zur Verengung 50 hin gehalten wird. Insbesondere kann die Verengung 50 so ausgestaltet sein, daß sie eine köcherartige Halterung für den in der Vertiefung 47 abgelegten Antriebsmotor bildet. Zusätzlich kann jedoch auch eine entsprechende Verengung oder köcherartige Halterung auf der zu der Verengung 50 entgegengesetzt liegenden Seite der Vertiefung 47 angeordnet sein.

Gemäß Fig. 6 sind symmetrisch und im wesentlichen parallel zu der Vertiefung 47 zwei schalenförmige Ablagen 48 und 49 angeordnet, die zur Aufnahme eines Behandlungswerkzeugs oder Behandlungsinstrumentes dienen. Gemäß Fig. 6a weisen diese Ablageabschnitte 48 und 49 eine längliche Form auf, wobei diese Ablageabschnitte 48 und 49 jedoch auch an die Form eines zahnärztlichen Handstücks oder Winkelstücks angepaßt sein können. Des weiteren können natürlich auch mehrere dieser Ablageabschnitte 48 und 49 vorgesehen sein. Wie bei der Vertiefung 47 ist auch die Tiefe der schalenförmigen Ablageabschnitte 48 und 49 vorzugsweise so gewählt, daß ein darin abgelegtes Behandlungsinstrument teilweise von der Oberfläche der Ablage 24 hervorsteht, um ein Entnehmen des Behandlungsinstruments zu erleichtern.

Die Seitenwand 46 der in Fig. 6 gezeigten Ablage 24 wird durch einen oberen Rand 44, der durch die Vertiefung 47 unterbrochen wird, sowie einen umlaufenden unteren Rand 45 begrenzt. Wie insbesondere aus Fig. 6b ersichtlich ist, steht der untere Rand aus noch nachfolgend näher zu erläuternden Gründen etwas weiter von der Seitenwand 46 ab. Der obere Rand 44, der untere Rand 45 und die dazwischen angeordnete Seitenwand 46 bestimmen die im wesentlichen ellipsenartige Form der in Fig. 6 gezeigten Ablage 24 und bilden eine Aufnahmeanordnung für den in Fig. 1 gezeigten Versorgungsschlauch 22, da dieser zwischen die beiden Ränder 44 und 45 um die Seitenwand 46 gewickelt werden kann und dabei von den beiden Rändern 44 und 45 gehalten wird. Anstelle der in Fig. 6a dargestellten nahezu vollständig ausgebildeten umlaufenden Form des oberen Randes 44 würde es auch genügen, wenn der obere Rand 44 lediglich teilweise an beispielsweise zwei gegenüberliegenden Stellen der Ablage 24 ausgebildet ist, wodurch ebenfalls bereits eine sichere Halterung des um die Seitenwand 46 gewickelten Versorgungsschlauches gewährleistet wäre.

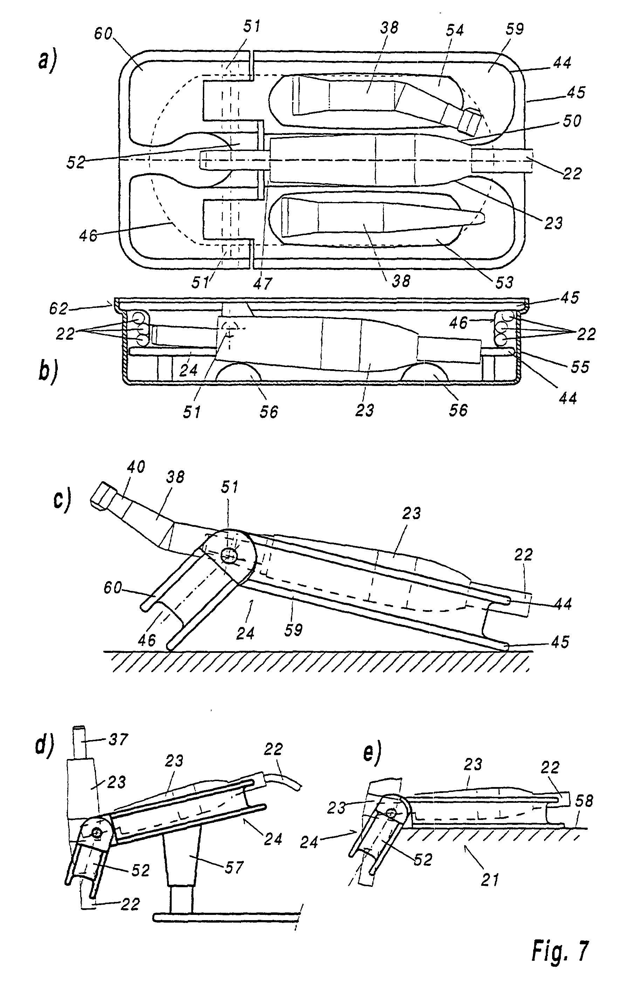

Fig. 7 zeigt in verschiedenen Ansichten ein zweites Ausführungsbeispiel der in Fig. 1 dargestellten Ablage 24, wobei es sich bei diesem Ausführungsbeispiel um eine zweiteilige Ausführungsform handelt. Fig. 7a zeigt eine Draufsicht auf diese Ablage, wobei erkennbar ist, daß zwei Ablageteile 59 und 60 gelenkartig durch entsprechende Gelenkmittel 51 miteinander verbunden sind, so daß insbesondere der Ablageteil 60 gegenüber dem Ablageteil 59 geschwenkt werden kann. Der Ablageteil 59 weist Ablageabschnitte 53 und 54 für die Aufnahme von Behandlungsinstrumente 38 auf, wobei gemäß Fig. 7 beispielhaft hinsichtlich des Ablageabschnitts 54 ein Winkelstück und hinsichtlich des Ablageabschnitts 53 ein Handstück dargestellt ist. Zudem ist in dem Ablageteil 59 die bereits anhand von Fig. 6 beschriebene Vertiefung 47 für den Antriebsmotor 23 mit der Verengung 50 enthalten. Der gegenüber dem Ablageteil 59 schwenk- bzw. drehbare Ablageteil 60 weist eine der Vertiefung 47 im wesentlichen entsprechende Vertiefung 52 auf, wobei diese jedoch in Form einer Köcherhalterung für den Antriebsmotor 23 ausgestaltet ist, so daß der Antriebsmotor 23 einerseits in der Vertiefung 47 abgelegt und andererseits in der köcherartigen Halterung 52 nach Schwenkung des Ablageteils 60 gegenüber dem Ablageteil 59 gehalten werden kann.

Dies ist insbesondere in Fig. 7d und 7e dargestellt, wobei Fig. 7d beispielhaft die Plazierung der Ablage 24 auf einen zu dem in Fig. 1 dargestellten Steuergerät 21 gehörenden Ablagearm 57 darstellt. Fig. 7e zeigt beispielhaft direkt die Auflage der Ablage 24 auf einer Oberfläche 58 des Steuergeräts 21. Sowohl in Fig. 7d als auch in Fig. 7e ist jeweils der Antriebsmotor 23 einerseits in einer in der Vertiefung 47 abgelegten Stellung und andererseits in einer in der Köcherhalterung 52 gehaltenen nahezu senkrechten Stellung dargestellt.

Die zweiteilige Ausführungsform der Ablage 24 weist den Vorteil auf, daß - wie in Fig. 7c gezeigt ist - durch Schwenken des Ablageteils 60 gegenüber dem Ablageteil 59 auf einfache Weise eine gewünschte Neigung der Ablage 24 stufenlos eingestellt werden kann, wobei die Reibung zwischen den in Fig. 7c gezeigten Gelenkmitteln und den beiden Ablageteilen 59 und 60 einerseits so klein ist, daß die Ablageteile 59 und 60 gegeneinander geschwenkt werden können, und andererseits jedoch groß genug ist, daß auch nach Ablage des Antriebsmotors 23 sowie der Behandlungsinstrumente 38 in der Ablage 24 die gemäß Fig. 7c herbeigeführte Stellung der Ablage 24 beibehalten werden kann.

Wie bei dem in Fig. 6 gezeigten Ausführungsbeispiel steht auch bei dem in Fig. 7 dargestellten Ausführungsbeispiel der untere Rand 45 weiter als der obere Rand 44 von der Seitenwand 46 seitlich ab. Dadurch kann die Ablage 24 in eine in Fig. 7b gezeigte Sterilisationskassette 55 eingesetzt werden, wobei die Ablage 24 in der Sterilisationskassette 55 mit dem Rand 44 nach unten gerichtet abgelegt ist. Ein in der Ablage gehaltener Antriebsmotor 23 kommt dabei auf entsprechenden Auflageflächen 56 zu liegen. Der weiter von der Seitenwand 46 der Auflage 24 hervorstehende Rand 45 liegt dabei auf Schultern 62 der Sterilisationskassette 55 auf, so daß die Ablage 24 zugleich als "Verpackung" für die in der Ablage 24 gehaltenen Instrumente bzw. den Antriebsmotor 23 und den Versorgungsschlauch 22 dient. Aus Fig. 7b ist insbesondere auch der auf die Seitenwand 46 der Ablage 24 aufgewickelte Versorgungsschlauch 22 ersichtlich.

Bei der in Fig. 7b dargestellten Sterilisationskassette 55 handelt es sich um einen mehrfach sterilisierbaren Behälter, in den durch entsprechend vorgesehene Öffnungen Sterilisierdampf bzw. Sterilisiergas zur Sterilisierung der Ablage 24 und der darin gehaltenen Instrumente, des Antriebsmotors 23 und des Versorgungsschlauches 22 eingeführt werden kann. Eine derartige Sterilisationskassette ist beispielsweise aus der EP-A1-0588 228 oder der DE-U1-92 06 022 bekannt. In der Regel erfolgt die Sterilisation bei einer Temperatur von bis zu 135° C. Während beim bekannten Stand der Technik die einzelnen zu sterilisierenden Teile in einen Klarsichtbeutel verpackt werden müssen, können die in der in Fig. 6 und 7 gezeigten Ablage 24 abgelegten Teile zusammen mit der Ablage 24 in einer entsprechenden Sterilisationskassette sterilisiert werden, ohne daß eine Verpackung in Klarsichtbeutel erforderlich ist. Zudem können die einzelnen Behandlungsinstrumente bequem zusammen mit dem Antriebsmotor und dem Versorgungsschlauch sterilisiert werden. Allgemein ist die Ablage 24 derart geformt, daß sie in eine übliche Sterilisationskassette, Sterilisationstray oder einen üblichen Sterilisationskorb vorzugsweise schlüssig eingesetzt werden kann.

Nachdem die zu sterilisierenden Teile zusammen mit der Ablage 24 sterilisiert werden, muß die Ablage 24 aus einem sterilisierbaren Material, wie z.B. Silikon, hergestellt sein. Die Ablage 24 ist frei positionierbar und kann durch Befestigungsmittel an entsprechenden Stellen des in Fig. 1 gezeigten Steuergerätes 21 oder - wie in Fig. 7d angedeutet - auf einem Ablagearm 57 des Steuergeräts befestigt werden.

Insbesondere bei Anwendungen in der Implantologie sind zum Teil sehr genaue Drehmomente erforderlich. Diese Drehmomente sind nicht allein vom Strom des Antriebsmotors abhängig, sondern auch vom Zustand des mit dem Antriebsmotor gekoppelten Behandlungsinstrumentes. Es wird daher vorgeschlagen, für eine Drehmomentgenauigkeit von kleiner ± 3% den Antriebsmotor mit darauf aufgesetztem Behandlungsinstrument zu kalibrieren. Bei der Drehmomentkalibrierung wird das tatsächlich von dem Antriebsmotor und dem damit gekoppelten Behandlungsinstrument ausgeübte Drehmoment bzw. die entsprechende Drehmomentkennlinie ermittelt und anschließend der Antriebsmotor mit dem daran gekoppelten Behandlungsinstrument gemäß dem ermittelten Drehmoment bzw. der ermittelten Drehmomentkennlinie angesteuert, so daß der Betrieb des Antriebsmotors und des Behandlungsinstruments durch die Drehmomentkalibrierung auf das tatsächlich von dem Antiebsmotor und dem Behandlungsinstrument ausgeübte Drehmoment abgestimmt ist.

Die Drehmomentkalibrierung bzw. die Ermittlung des Drehmoments erfolgt mit Hilfe einer Drehmomentermittlungseinrichtung, die einerseits in das in Fig. 1 gezeigte Steuergerät 21 integriert oder andererseits als eine an das Steuergerät 21 anschließbare separate Einheit vorgesehen sein kann. Vorteilhafterweise umfaßt die Drehmomentermittlungseinrichtung ein Softwaremodul mit Bedienerführung, mit dessen Hilfe auf einer der Drehmomentermittlungseinrichtung zugeordneten Anzeigeneinheit oder auf der Anzeigeneinheit 36 des Steuergeräts 21 entsprechende Ablauf- bzw. Bedieninformationen für die Durchführung der Drehmomentkalibrierung angezeigt werden.

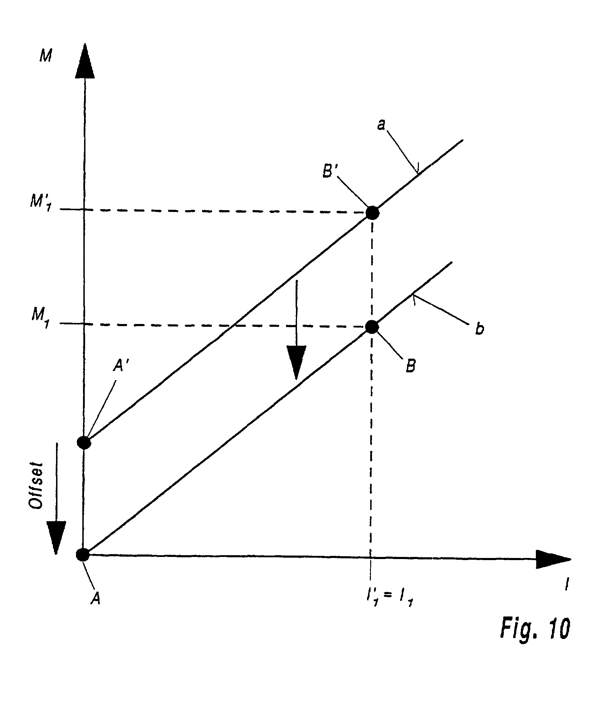

Zur Durchführung der Drehmomentkalibrierung muß zunächst im Falle einer externen Drehmomentermittlungseinrichtung diese Drehmomentermittlungseinrichtung an das Steuergerät 21 angeschlossen und anschließend - wie bereits anhand von Fig. 2 erläutert worden ist - die in Fig. 2 gezeigte Taste 12 betätigt werden, um die Drehmomentkalibrierungsprozedur zu starten. Anschließend wird mit Hilfe der zuvor beschriebenen Bedienerführung der Benutzer zur Durchführung der nachfolgend näher anhand von Fig. 10 beschriebenen Drehmomentkalibrierungsprozedur aufgefordert.

Fig. 10 zeigt zwei Drehmoment-Motorstrom-Kennlinen a und b. Die Kennlinie a zeigt die sich bei Kopplung eines Behandlungsinstruments mit dem Antriebsmotor ergebende Kennlinie ohne Drehmomentkalibrierung. Mit Hilfe der Drehmomentkalibrierung soll nunmehr die Ist-Kennlinie a zu der Soll-Kennlinie b verschoben werden. Die Soll-Kennlinie b entspricht derjenigen Kennlinie, mit der das in Fig. 1 und 2 gezeigte Steuergerät 21 normalerweise den Antriebsmotor mit dem daran gekoppelten Behandlungsinstrument ansteuern würde. Die Soll-Kennlinie b ist demnach dem Steuergerät 21 bekannt. Zur Eliminierung des in Fig. 10 gezeigten Offset zwischen den Kennlinien a und b genügt es somit, einen bestimmten Meßpunkt B' der Ist-Kennlinie a zu ermitteln, so daß aufgrund des Unterschieds zwischen dem dem Meßpunkt B' zugeordneten Drehmoment M'₁ und dem dem Soll-Meßpunkt B' zugeordneten Drehmoment M₁ auf die Verschiebung zwischen den beiden Kennlinien a und b geschlossen werden kann, um diese anschließend zu eliminieren. Bei der Eliminierung dieses Offset wird die Ist-Kennlinie a zu der Soll-Kennlinie b verschoben, so daß der im Leerlauf des Antriebsmotors (I = 0) auftretende Drehmomentwert A' zum Meßpunkt A verschoben und somit als neuer Nullpunkt behandelt wird.

Zur Ermittlung des in Fig. 10 dargestellten Meßpunktes B' muß zum einen der entsprechende Drehmomentwert M'₁ und zum anderen der Motorstromwert I'₁ ermittelt werden. Der Motorstrom kann beispielsweise wie in der DE-A1-196 28 854 mit Hilfe eines in Serie mit dem Antriebsmotor geschalteten Widerstands erfaßt werden, wobei die an dem Widerstand abfallende Spannung als proportionale Größe zu dem Motorstrom ausgewertet wird.

Anhand Fig. 10 wurde der Fall erläutert, daß die Ist-Kennlinie a parallel zu der Soll-Kennlinie b verläuft. Unter bestimmten Umständen kann jedoch die Ist-Kennlinie a auch geneigt zu der Soll-Kennlinie b verlaufen. In diesem Fall müssen zur Ermittlung der Ist-Kennlinie a zwei Meßpunkte ermittelt werden, die die Ist-Kennlinie a eindeutig definieren. Vorteilhafterweise ist eine der beiden Meßpunkte der Meßpunkt A' im Leerlauf des Antriebsmotors (I = 0), so daß aufgrund des dem Meßpunkt A' entsprechenden Drehmomentwertes die Ist-Kennlinie in den Nullpunkt verschoben werden kann.

Allgemein kann z.B. vorgesehen sein, daß die Drehmomentermittlungseinrichtung den Motorstrom bzw. die Motorleistung standardmäßig für z.B. drei verschiedene Drehmomentwerte, wie z.B. 0 Ncm (Leerlauf), 5 Ncm und 35 Ncm, mißt, um daraus die für die Kalibrierung erforderlichen Drehmomentdaten zu gewinnen. Liegt ein Meßwert außerhalb vorgegebener Meßwertgrenzen, kann die Bedienperson über die Anzeige 36 zur Überprüfung des Antriebsmotors 23 bzw. des darauf aufgesetzten Behandlungsinstrumentes 38 oder zur Wiederholung der Drehmomentkalibrierung aufgefordert werden.

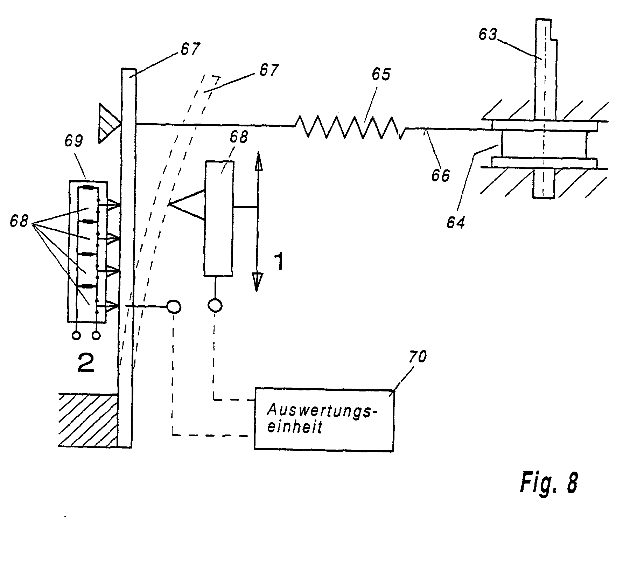

Fig. 8 zeigt ein erstes Ausführungsbeispiel einer Drehmomentermittlungseinrichtung, mit der das tatsächlich von dem Antriebsmotor und dem darauf aufgesetzten Behandlungsinstrument ausgeübte Drehmoment ermittelt werden kann. Gemäß Fig. 8 wird das von dem Antriebsmotor sowie dem Behandlungsinstrument ausgeübte Drehmoment in eine Verstellkraft für ein Verstellmedium umgesetzt, wobei der von der Verstellkraft hervorgerufene Verstellweg des Verstellmediums erfaßt und in ein entsprechendes elektrisches Signal umgewandelt wird. Bei dem in Fig. 8 gezeigten Ausführungsbeispiel ist das Verstellmedium eine einseitig eingespannte und vorgespannte Blattfeder 67. Das von dem Antriebsmotor und dem Behandlungsinstrument ausgeübte Drehmoment wird von einem Mitnehmer bzw. einer Aufnahmewelle 63 aufgenommen, welche mechanisch mit einer Wickeltrommel 64 gekoppelt ist. Die Wickeltrommel 64 ist über ein Spannseil 66 mit dem nicht eingespannten Ende der Blattfeder 67 verbunden. In das Spannseil ist eine Feder 65 zur Dämpfung und Verringerung des Spannweges eingesetzt, die zudem eine Kraftvorspannung des Seils ermöglicht.

Bei Ausübung eines bestimmten Drehmoments des Antriebsmotors mit dem daran gekoppelten Behandlungsinstrument wird die Aufnahmewelle 63 gedreht und das Seil 66 entsprechend auf der Wickeltrommel 64 aufgewickelt. Bei einer bestimmten, geeichten Kraft verläßt die Blattfeder 67 ihre ursprüngliche Position und wird in die in Fig. 8 gestrichelt gezeichnete Auslenkposition bewegt. Diese Auslenkung bzw. dieser Verstellweg ist ein Maß für das auf die Aufnahmewelle 63 ausgeübte Drehmoment. Sobald an der Aufnahmewelle 63 kein Drehmoment mehr anliegt, bewegt sich die Blattfeder 67 aufgrund ihrer Vorspannung zurück in ihre Ausgangsposition und das Seil 66 wird wieder von der Wickeltrommel 64 abgewickelt.

Zur Erfassung des Verstellweges, d.h. der Auslenkung, der Blattfeder 67 ist eine Schaltanordnung 68 vorgesehen, wobei gemäß Fig. 8 zwischen zwei Varianten 1 und 2 unterschieden wird. Gemäß der Variante 1 ist ein Schalter 68 vorgesehen, der zusammen mit der Blattfeder 67 sowie einer Auswertungseinheit 70 einen Stromkreis bildet. Bei einer bestimmten geeichten Verstellkraft des Seils 66 wird der Schalter 68 betätigt und dadurch eine Signaländerung ausgelöst, die der Auswertungseinheit 70 zugeführt und mitgeteilt wird. Der Schalter 68 kann fest oder aber auch insbesondere entsprechend den Meßwerten in Pfeilrichtung verschiebbar angeordnt werden. Durch Verschieben des Schalters 68 nach oben kann demnach ein kleineres Drehmoment und durch Verschieben des Schalters 68 nach unten ein größeres Drehmoment erfaßt werden. Die Auswertungseinheit 70 setzt das von dem Schalter 68 gelieferte Signal bzw. die entsprechende Signaländerung mit Hilfe einer entsprechenden Software in das zu messende Drehmoment um. Mit Hilfe des von der Auswertungseinheit 70 gelieferten Drehmoments ist somit eine Korrektur der in Fig. 10 gezeigten Ist-Kennlinie a möglich, und der Antriebsmotor mit dem daran gekoppelten Behandlungsinstrument kann mit größtmöglicher Genauigkeit unter Berücksichtigung des in Fig. 10 dargestellten Offset betrieben werden. Die in Fig. 8 gezeigte Auswertungseinheit 70 kann in der Drehmomentermittlungseinrichtung oder aber auch in dem in Fig. 1 und 2 gezeigten Steuergerät 21 der ärztlichen Behandlungsvorrichtung enthalten sein.

Gemäß der durch 2 in Fig. 8 bezeichneten zweiten Variante der Schalteranordnung 68 sind mehrere einzelne Schalter in Längsrichtung der Blattfeder 67 vorgesehen, die jeweils mit entsprechenden Widerständen 69 gekoppelt sind und somit ein Schalter-Widerstandsnetzwerk bilden. Abhängig von der Auslenkung der Blattfeder 67 werden somit unterschiedliche Schalter dieser Schalteranordnung betätigt, so daß an den Ausgangsanschlüssen dieser Schalteranordnung abhängig von der Auslenkung der Blattfeder 67 ein unterschiedlicher Gesamt-Widerstandswert auftritt, der von der Auswertungseinheit 70 ausgewertet und in das entsprechende Drehmoment umgerechnet werden kann.

Anstelle der in Fig. 8 gezeigten Schalteranordnung kann beispielsweise auch ein Potentiometer verwendet werden, dessen Schleifkontakt mit der Blattfeder 67 mechanisch gekoppelt ist, so daß sich das von dem Potentiometer gelieferte Ausgangssignal proportional mit der Auslenkung der Blattfeder 67 verändert. Das Ausgangssignal des Potentiometers wird dabei ebenfalls der in Fig. 8 gezeigten Auswertungseinheit 70 zugeführt.

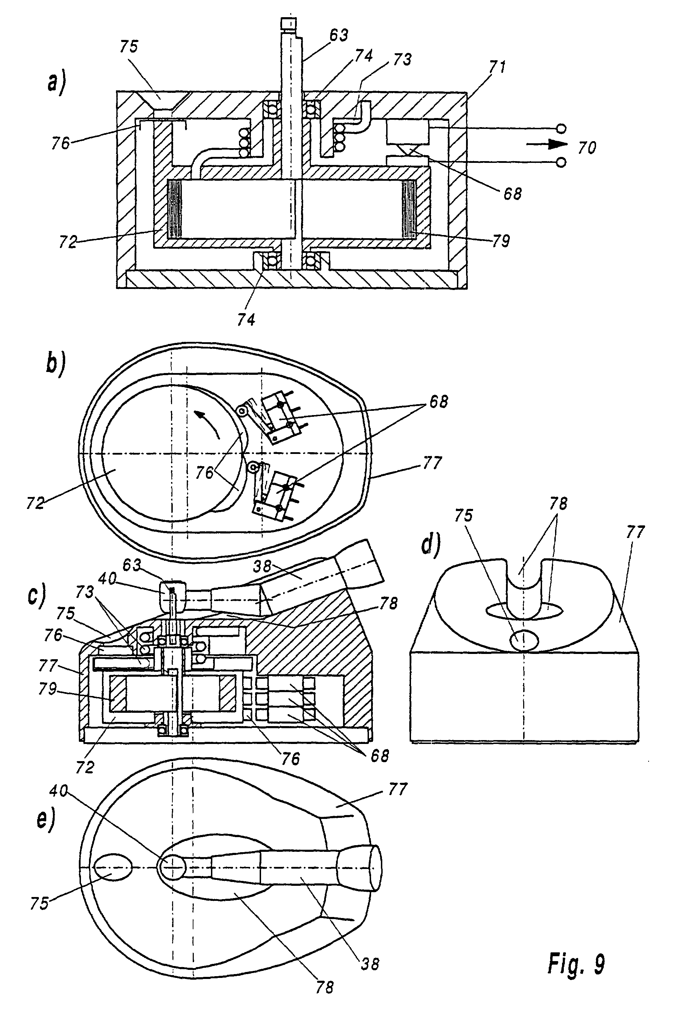

Fig. 9 zeigt verschiedene Ansichten eines weiteren Ausführungsbeispiels der zuvor beschriebenen Drehmomentermittlungseinrichtung, welche im Sinne einer Hysteresebremse realisiert ist. Fig. 9a zeigt eine Querschnittsansicht dieser Einrichtung, wobei ebenfalls eine Aufnahmewelle 63 zur Aufnahme bzw. Übertragung des von dem Antriebsmotor mit dem Behandlungsinstrument ausgeübten Drehmoments vorgesehen ist. Die Welle 63 ist mit einem durch Kugellager 74 in einem Außengehäuse 71 drehbar gelagerten Gehäuse- bzw. Drehkörper 72 verbunden, wobei dieser Drehkörper 72 über eine Spiralfeder 73 mit dem Außengehäuse 71 gekoppelt ist. Bei Ausübung eines Drehmoments auf den Mitnehmer bzw. die Aufnahmewelle 63 wird dieser Drehkörper 72 in dem Außengehäuse 71 verdreht, und es werden analog zu dem in Fig. 8 gezeigten Ausführungsbeispiel abhängig von dem Verdrehwinkel entsprechend angeordnete Schaltermittel 68 aktiviert, deren Ausgangssignale der in Fig. 8 gezeigten Auswertungseinheit 70 zugeführt werden, die abhängig von den Signalveränderungen der Schaltermittel das entsprechende Drehmoment bestimmt. In dem Drehkörper 72 ist eine Flachspiralfeder 79 mit beispielsweise 20 oder 40 Wicklungen vorhanden, die sich bei Verdrehen der Aufnahmewelle 63 auf der Aufnahmewelle 63 abwickelt und somit eine Rückstellkraft für den Drehkörper 72 ausübt, um diesen wieder in die ursprüngliche Position zurückzubewegen, wenn kein Drehmoment mehr auf die Aufnahmewelle 63 ausgeübt wird. Das Außengehäuse 71 besitz ein Fenster 75, so daß durch dieses Fenster eine Ableseskala bzw. Analoganzeige 76 des Drehkörpers 72 beobachtet werden kann. Vorzugsweise weist die Analoganzeige 76 direkt die der Verdrehung des Drehkörpers 72 entsprechenden Drehmomentwerte auf, so daß der Benutzer unmittelbar abhängig von der Verdrehung des Drehkörpers 72 das entsprechende Drehmoment ablesen kann.

Fig. 9b - 9e zeigen verschiedene Ansichten einer Variante des in Fig. 9a gezeigten Ausführungsbeispiels, wobei Fig. 9c die entsprechende Querschnittsansicht, Fig. 9d eine Frontalansicht und Fig. 9e eine Draufsicht dieser Variante darstellt. Fig. 9b zeigt eine Draufsicht auf den Innenraum der in Fig. 9c dargestellten Variante mit den wesentlichen Bauteilen.

Die in Fig. 9c dargestellten Elemente entsprechen im wesentlichen den in Fig. 9a dargestellten Elementen und sind mit entsprechenden Bezugszeichen versehen. Die Drehmomentermittlungseinrichtung ist jedoch anstelle des in Fig. 9a gezeigten Gehäuses in einen Gehäusekörper 77 eingebaut, der zudem eine Ausnehmung bzw. eine Ablagenut 78 für ein Behandlungsinstrument, gemäß Fig. 9c ein Winkelstück 38 mit daran gekoppeltem Kopfteil 40 aufweist, wobei das Behandlungsinstrument 38 in der Ausnehmung 78 abgelegt und zugleich in dieser Ablagestellung mechanisch mit der Aufnahmewelle 63 der Drehmomentermittlungseinrichtung gekoppelt werden kann. Aus Fig. 9c in Verbindung mit Fig. 9b ist zudem ersichtlich, daß die Außenseite des Drehkörpers 72 mehrere hervorstehende Schaltnocken 76 aufweist, die bei Drehung des Drehkörpers 72 in der in Fig. 9b dargestellten Pfeilrichtung entsprechend angeordnete Schalter abhängig von dem Verdrehwinkel des Drehkörpers 72 betätigen. Wie in Fig. 9c gezeigt ist, können mehrere dieser Schaltnocken 76 sowie die entsprechenden Schalter 68 vertikal übereinanderliegend angeordnet sein. Vorzugsweise sind die Schaltnocken mit der in Fig. 9b gezeigten Form gleichmäßig entlang des Umfangs des Drehkörpers 72 verteilt. Selbstverständlich können die Schaltnocken auch eine andere Außenform besitzen, soweit dadurch eine Betätigung der Schalter 68 möglich ist. Die Ausgangssignale der Schalter 68 werden wiederum der in Fig. 8 gezeigten Auswertungseinheit zugeführt, die abhängig von den von den Schaltern 68 gelieferten Signalveränderungen auf das auf die Aufnahmewelle 63 ausgeübte Drehmoment des Antriebsmotors mit dem daran gekoppelten Behandlungsinstrument schließt.

Zur Vorgabe bestimmter Drehmomentwerte können für die Aufnahmewelle 63 bzw. den Drehkörper 72 entsprechende Raststufen vorgesehen sein, die durch in Umfangsrichtung der Aufnahmewelle 63 bzw. des Drehkörpers 72 angeordnete (nicht gezeigte) Kugelraststifte realisiert sein können. Jede Raststufe entspricht dabei einem bestimmten Drehmoment, wie z.B. 0 Ncm, 15 Ncm oder 30 Ncm. Durch Einrasten der Aufnahmewelle 63 mit dem aufgesetzten Behandlungswerkzeug 38 bzw. des Drehkörpers 72 in die entsprechenden Raststufen kann der dem jeweiligen vorgegebenen Drehmoment entsprechende Motorstrom gemessen werden, um somit die gewüschten Drehmomentkennliniendaten gewinnen zu können.

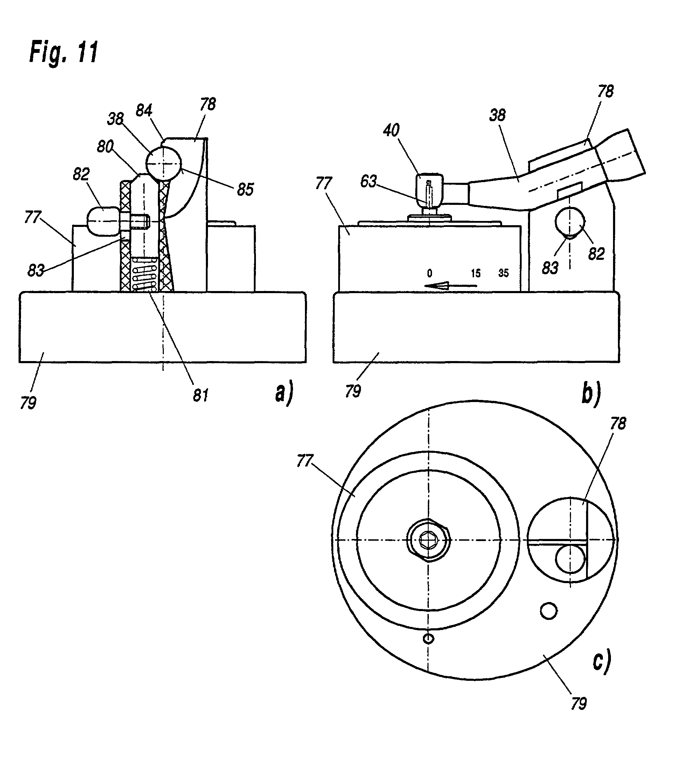

In Fig. 11 ist ein drittes Ausführungsbeispiel einer Drehmomentermittlungseinrichtung der vorliegenden Erfindung in verschiedenen Ansichten dargestellt, wobei Fig. 11b eine Seitenansicht, Fig. 11c eine Draufsicht und Fig. 11a eine Teilschnittansicht auf die rechte Längsseite der in Fig. 11b gezeigten Einrichtung zeigt. In Fig. 11a und 11b ist ein in einer Ablage 78 gehaltenes Winkelstück 38 mit Kopfteil 40 gezeigt. Das Kopfteil 40 ist wiederum zur Drehmomentermittlung mit einer Aufnahmewelle 63 zu koppeln. Auf die Darstellung des Winkelstücks 38 wurde in Fig. 11c verzichtet.

Auch das in Fig. 11 gezeigte Ausführungsbeispiel verwendet zur Drehmomentermittlung eine Hysterese-Bremse, die wiederum in einem Gehäuse 77 untergebracht ist. Das Hysterese-Bremsengehäuse 77 ist auf einen Sockel 79 aufgesetzt. In der Hysterese-Bremse sind, wie bereits zuvor erläutert worden ist, mehrere Rastbohrungen mit Federrastung integriert, die unterschiedlichen Auslenkungen des Drehkörpers der Hysterese-Bremse entsprechen, so daß der Drehkörper abhängig von seiner Auslenkung in Pfeilrichtung (vgl. Fig. 11b) in einer der Rastbohrungen einrastet. Gemäß der Darstellung in Fig. 11b entsprechen dabei in diesem Fall die Raststufen einem Drehmoment von 0 Ncm, 15 Ncm bzw. 35 Ncm.

Die Besonderheit des in Fig. 11 gezeigten Ausführungsbeispiels ist die Ausgestaltung der Ablage bzw. Halterung 78 für das Hand- oder Winkelstück 38. Wie insbesondere der Darstellung von Fig. 11a entnommen werden kann, umfaßt diese Halterung 78 eine Ausnehmung 85, die teilweise das Winkelstück 38 umgibt. Insbesondere kann diese Ausnehmung 85 im wesentlichen halbkreisförmig ausgestaltet sein. Die Ausnehmung 85 ist derart ausgestaltet, daß oben an dem Winkelstück 38 ein Vorsprung 84 anliegt. Des weiteren umfaßt die Halterung 78 einen durch eine Spiralfeder 81 federnd vorgespannten und bewegbar gelagerten Verriegelungsbolzen 80. Zur Ablage des Winkelstücks 38 wird das Winkelstück 38 gegen den Widerstand des Verriegelungsbolzens 80 in die Ausnehmung 85 gedrückt, so daß in der in Fig. 11a und 11b gezeigten Ablagestellung das Winkelstück in der Ausnehmung 85 durch die Federkraft der Spiralfeder 81 mittels des Verriegelungsbolzens nach oben gegen den Vorsprung 84 gedrückt und somit fixiert wird. Mit dem Verriegelungsbolzen 80 ist ein Anschlagbolzen 82 gekoppelt, der in Längsrichtung des Verriegelungsbolzens 80 in einem Langloch 83 verschiebbar gelagert ist und in der in Fig. 11a gezeigten Verriegelungsstellung an der Oberkante des Langlochs 83 anliegt, so daß ein Herausrutschen des Verriegelungsbolzens 80 verhindert wird. Zu Entriegelung des Winkelstücks 38 ist es lediglich erforderlich, den Anschlagbolzen 82 und damit den Verriegelungsbolzen 80 entgegen der Federkraft der Feder 81 nach unten zu drücken, so daß das Winkelstück 38 wieder aus der Ausnehmung 85 der Ablage bzw. Halterung 78 entnommen werden kann.

Bei jedem der in Fig. 8, 9 und 11 gezeigten Ausführungsbeispiele liefert die Auswertungseinheit 70 der entsprechenden Drehmomentermittlungseinrichtung das tatsächlich von dem Behandlungsinstrument und dem Antriebsmotor auf die Aufnahmewelle 63 ausgeübte Drehmoment bzw. ein diesem Drehmoment entsprechendes Signal, welches dem Steuergerät 21 bzw. einer in dem Steuergerät 21 vorhandenen Steuereinheit zugeführt wird, so daß das Steuergerät abhängig von dem somit ermittelten Drehmomentwert die Drehmomentkalibrierung, d.h. die anhand von Fig. 10 erläuterte Drehmomentkorrektur, durchführen und das Behandlungsinstrument mit größtmöglicher Genauigkeit ansteuern kann, indem der Antriebsmotor 23 mit einer entsprechenden Leistungszufuhr betrieben wird.

Bei den zuvor beschriebenen Drehmomentermittlungseinrichtungen wurde davon ausgegangen, daß das von einem auf den Antriebsmotor 23 aufgesetzten Behandlungsinstrument 38 ausgeübte Drehmoment ermittelt wird (z.B. mit Hilfe der Aufnahmewelle 63). Alternativ kann selbstverständlich auch eine Abwandlung dahingehend erfolgen, daß das Behandlungsinstrument 38 mit einem Behandlungswerkzeug 39 (z.B. einem Bohrer) gekoppelt ist und das von dem auf das Behandlungsinstrument 38 aufgesetzten Bohrer 39 ausgeübte Drehmoment ermittelt und zur Grundlage der Kalibrierung gemacht wird. Zudem besteht die Möglichkeit, daß die Drehmomentermittlungseinrichtung direkt, d.h. ohne die Hilfe des Steuergeräts 21, abhängig von den ermittelten Drehmomentdaten die Leistungszufuhr des Antriebsmotors 23 entsprechend einstellt.

Abschließend sei erwähnt, daß das in Fig. 1 und 2 gezeigte Steuergerät 21 der ärztlichen Behandlungsvorrichtung 20 auch einen Druckeranschluß aufweisen kann, so daß verschiedene technische Informationen, wie z.B. die auf der Anzeigeneinheit 36 dargestellten Betriebsparameterinformationen, das Datum, die Uhrzeit usw. ausgedruckt werden können. Anhand eines derartigen Ausdrucks kann anschließend der Behandlungsablauf nachvollzogen und beurteilt werden. Ebenso kann eine serielle oder parallele Schnittstelle zum Anschluß des Steuergeräts an einen Computer vorgesehen sein, um Daten an den Computer übertragen zu können und am Bildschirm des Computers eine vergrößerte Displayanzeige darstellen bzw. mit Hilfe des Computers Daten aufzeichnen zu können.

## Patentansprüche

1. Ärztliche Behandlungsvorrichtung (20)
mit einem Steuergerät (21)
mit einem an das Steuergerät (21) anschließbaren Antriebsmotor (23), mit dem ein Behandlungsinstrument (38) zu betreiben ist, und
mit einer Drehmomentermittlungseinrichtung, welche vor Inbetriebnahme des Antriebsmotors (23) sowie des damit gekoppelten Behandlungsinstruments (38) das tatsächlich von dem Antriebsmotor (23) und dem Behandlungsinstrument (38) ausgeübte Drehmoment bzw. die entsprechende Drehmomentkennlinie erfasst,
**dadurch gekennzeichnet,**
**dass** die Drehmomentermittlungseinrichtung
- Mitnahmemittel (63) zur Kopplung mit dem Behandlungsinstrument (38),
- Erfassungsmittel (63-69; 68, 72, 73, 75, 76), die ein Signal erzeugen, das dem tatsächlich von dem Antriebsmotor (23) und dem Behandlungsinstrument (38) auf die Mitnahmemittel (63) ausgeübten Drehmoment entspricht, sowie
- Auswertungsmittel (70), die dieses Signal bzw. die entsprechenden Drehmomentdaten an das Steuergerät (21) liefert,
aufweist,
**dass** das Steuergerät (21) abhängig von diesem Signal die Drehmomentkalibrierung, d.h. Drehmomentkorrektur durchführt und
**dass** das Steuergerät (21) nach dem Drehmomentkalibrierungsvorgang den Antriebsmotor (23) mit einer entsprechenden Leistungszufuhr betreibt.

2. Ärztliche Behandlungsvorrichtung nach Anspruch 1,
**dadurch gekennzeichnet,**
**daß** die Mitnahmemittel durch eine mit dem Behandlungsinstrument (38) koppelbare Aufnahmewelle (63) zur mechanischen Übertragung und Erfassung der Drehung eines rotierenden Antriebs des Behandlungsinstruments (38) gebildet sind.

3. Vorrichtung nach Anspruch 2,
**dadurch gekennzeichnet,**
**daß** die mit dem Behandlungsinstrument koppelbare Aufnahmewelle (63) eine Hysterese-Bremse antreibt.

4. Ärztliche Behandlungsvorrichtung nach einem der Ansprüche 1 bis 3,
**dadurch gekennzeichnet,**
**daß** die Drehmomentermittlungseinrichtung die Drehmomentdaten durch Korrelation des von ihr ermittelten Drehmoments des Behandlungsinstruments (38) mit der dem Antriebsmotor (23) zugeführten Leistung gewinnt.

5. Ärztliche Behandlungsvorrichtung nach einem der Ansprüche 1 bis 4,
**dadurch gekennzeichnet,**
**daß** die Mitnahmemittel (63) die Drehbewegung des Behandlungsinstruments (38) in eine Verstellkraft für ein Verstellmedium (67; 72) umsetzen und daß die Erfassungsmittel ferner Umwandlungsmittel (68, 69; 68) aufweisen, welche einen durch die Verstellkraft hervorgerufenen Verstellweg des Verstellmediums (67; 72) erfassen und davon abhängig das elektrische Signal liefern.

6. Ärztliche Behandlungsvorrichtung nach Anspruch 5,
**dadurch gekennzeichnet,**
**daß** das Verstellmedium (67; 72) federelastisch vorgespannt ist.

7. Ärztliche Behandlungsvorrichtung nach Anspruch 5 oder 6,
**dadurch gekennzeichnet,**
**daß** die Umwandlungsmittel (68, 69; 68) den Verstellweg des Verstellmediums (67; 72) durch eine Schalteranordnung erfassen, welche abhängig vom Verstellweg des Verstellmediums (67; 72) unterschiedliche elektrische Signale liefert.

8. Ärztliche Behandlungsvorrichtung nach Anspruch 7,
**dadurch gekennzeichnet,**
**daß** die Schalteranordnung (68, 69) mehrere Schalter umfaßt, welche abhängig vom Verstellweg des Verstellmediums (67; 72) unterschiedlich betätigt werden.

9. Ärztliche Behandlungsvorrichtung nach Anspruch 8,
**dadurch gekennzeichnet,**
**daß** die Schalteranordnung mehrere mit den Schaltern (68) kombinierte Widerstände (69) umfaßt, so daß die Schalteranordnung abhängig von der Betätigung der einzelnen Schalter einen von dem Verstellweg des Verstellmediums (67; 72) abhängigen unterschiedlichen Gesamtwiderstandswert aufweist.

10. Ärztliche Behandlungsvorrichtung nach Anspruch 5 oder 6,
**dadurch gekennzeichnet,**
**daß** die Umwandlungsmittel (68, 69; 68) ein Potentiometer aufweisen, dessen Widerstandswert sich abhängig von dem Verstellweg des Verstellmediums (67; 72) verändert und welches das elektrische Signal liefert.

11. Ärztliche Behandlungsvorrichtung nach Anspruch 2 und einem der Ansprüche 5 bis 10,
**dadurch gekennzeichnet,**
**daß** das Verstellmedium (67; 72) mit der Aufnahmewelle (63) mechanisch gekoppelt ist.

12. Ärztliche Behandlungsvorrichtung nach Anspruch 11,
**dadurch gekennzeichnet,**
**daß** das Verstellmedium eine an einem ersten Ende gehaltene Blattfeder (67) ist, deren zweites Ende mit der Aufnahmewelle(63) mechanisch gekoppelt ist, wobei die Umwandlungsmittel (68, 69) den Verstellweg des zweiten Endes der Blattfeder (67) erfassen.

13. Ärztliche Behandlungsvorrichtung nach Anspruch 12,
**dadurch gekennzeichnet,**
**daß** das zweite Ende der Blattfeder (67) über ein vorgespanntes Seil (66) mit der Aufnahmewelle (63) gekoppelt ist.

14. Ärztliche Behandlungsvorrichtung nach Anspruch 13,
**dadurch gekennzeichnet,**
**daß** in das Seil (66) ein federelastisches Mittel (65) eingesetzt ist, welches in Zugrichtung des Seils (66) gedehnt wird.

15. Ärztliche Behandlungsvorrichtung nach Anspruch 13 oder 14,
**dadurch gekennzeichnet,**
**daß** die Aufnahmewelle (63) mechanisch mit einer Wickeltrommel (64) gekoppelt ist, an der das Seil (66) befestigt ist, so daß sich das Seil (66) bei Übertragung einer Drehung von dem Behandlungsinstrument (38) auf die Aufnahmewelle (63) auf der Wickeltrommel (64) aufwickelt.

16. Ärztliche Behandlungsvorrichtung nach einem der Ansprüche 12 - 15 und Anspruch 8 oder 9,
**dadurch gekennzeichnet,**
**daß** die mehreren Schalter (68) in Längsrichtung der Blattfeder (67) angeordnet sind und somit abhängig von der Auslenkung des zweiten Endes der Blattfeder (67) unterschiedlich betätigt werden.

17. Ärztliche Behandlungsvorrichtung nach einem der Ansprüche 12 - 16 und einem der Ansprüche 5 - 7,
**dadurch gekennzeichnet,**
**daß** die Schalteranordnung (68, 69) entlang der Längsrichtung der Blattfeder (67) verschiebbar ist.

18. Ärztliche Behandlungsvorrichtung nach Anspruch 11,
**dadurch gekennzeichnet,**
**daß** das Verstellmedium ein mit der Aufnahmewelle (63) mechanisch gekoppelter Drehkörper (72) ist, dessen Auslenkung durch die Umwandlungsmittel (68) erfaßt wird.

19. Ärztliche Behandlungsvorrichtung nach Anspruch 18 und Anspruch 8 oder 9,
**dadurch gekennzeichnet,**
**daß** die Schalter (68) entlang der Umfangsrichtung des Drehkörpers (72) angeordnet sind und von Nocken (76) betätigt werden, welche von der Außenseite des Drehkörpers (72) seitlich hervorstehen.

20. Ärztliche Behandlungsvorrichtung nach Anspruch 19,
**dadurch gekennzeichnet,**
**daß** die Schalter (68) gleichmäßig entlang der Umfangsrichtung des Drehkörpers (72) verteilt angeordnet sind.

21. Ärztliche Behandlungsvorrichtung nach einem der Ansprüche 18 - 20,
**dadurch gekennzeichnet,**
**daß** elastische Rückstellmittel (73,79) vorgesehen sind, welche bei Nichtvorliegen eines Drehmoments des Behandlungsinstruments (38) die Aufnahmewelle und den Drehkörper (72) in eine Ausgangsstellung zurückstellen.

22. Ärztliche Behandlungsvorrichtung nach Anspruch 21,
**dadurch gekennzeichnet,**
**daß** die Rückstellmittel eine mit dem Drehkörper (72) mechanisch gekoppelte Spiralfeder (73) umfassen, welche die Aufnahmewelle (63) umgibt.

23. Ärztliche Behandlungsvorrichtung nach Anspruch 21 oder 22,
**dadurch gekennzeichnet,**
**daß** die Rückstellmittel eine mehrfach in dem Drehkörper (72) gewickelte Flachspiralfeder (79) umfassen, welche sich bei Auslenkung des Drehkörpers (72) infolge eines auf die Aufnahmewelle (63) übertragenen Drehmoments auf die Aufnahmewelle (63) aufwickelt.

24. Ärztliche Behandlungsvorrichtung nach einem der vorherigen Ansprüche,
**dadurch gekennzeichnet,**
**daß** die Drehmomentermittlungseinrichtung ein Gehäuse (71, 77) aufweist, welches eine Ablage (78) für das Behandlungsinstrument (38) aufweist, wobei ein rotierender Antrieb des in der Ablage (78) abgelegten Behandlungsinstruments (38) zugleich mit den Erfassungsmitteln (63 - 69; 63, 68, 72, 73, 75, 76) zur Erfassung des von dem rotierenden Antrieb des Behandlungsinstruments (38) ausgeübten Drehmoments mechanisch koppelbar ist.

25. Ärztliche Behandlungsvorrichtung nach Anspruch 24,
**dadurch gekennzeichnet,**
**daß** die Ablage (78) Fixiermittel (80-85) zum Fixieren des Behandlungsinstruments (38) in einer Ablageposition aufweist.

26. Ärztliche Behandlungsvorrichtung nach Anspruch 25,
**dadurch gekennzeichnet,**
**daß** die Fixiermittel (80-85) eine Ausnehmung (85) umfassen, in der das Behandlungsinstrument (38) abzulegen ist, wobei die Ausnehmung (85) einen Anschlagrand (84) aufweist, gegen den das Behandlungsinstrument (38) in der Ablageposition durch ein elastisch vorgespanntes Element (80) gedrückt und somit fixiert wird, wobei die Fixierung durch Verschieben des elastisch vorgespannten Elements (80) entgegen die Vorspannkraft lösbar ist.

27. Ärztliche Behandlungsvorrichtung nach einem der vorhergehenden Ansprüche,
**gekennzeichnet durch**
Anzeigemittel (75, 76) zur optischen Anzeige des von der Drehmomentermittlungseinrichtung ermittelten Drehmoments.

28. Ärztliche Behandlungsvorrichtung nach Anspruch 27 und einem der Ansprüche 24 bis 26,
**dadurch gekennzeichnet,**
**daß** die Anzeigemittel ein in dem Gehäuse (71, 77) ausgebildetes Fenster (75) aufweisen, durch welches der Verstellweg des Verstellmediums (72) überwacht werden kann.

29. Ärztliche Behandlungsvorrichtung nach einem der vorhergehenden Ansprüche,
**dadurch gekennzeichnet,**
**daß** die Drehmomentermittlungseinrichtung ein Steuermodul zur Benutzerführung aufweist.

30. Ärztliche Behandlungsvorrichtung nach einem der vorhergehenden Ansprüche,
**dadurch gekennzeichnet,**
**daß** die Drehmomentermittlungseinrichtung in das Steuergerät (21) integriert ist.

31. Verfahren zum Betreiben eines motorgetriebenen ärztlichen Behandlungsinstruments (38), wobei vor der Inbetriebnahme des Behandlungsinstruments (38) bei einer Drehmomentkalibrierung das tatsächlich von dem Antriebsmotor (23) und dem damit gekoppelten Behandlungsinstrument ausgeübte Drehmoment bzw. die entsprechende Drehmomentkennlinie ermittelt wird,
**dadurch gekennzeichnet,**
**dass**
- das Behandlungsinstrument (38) mit Mitnahmemitteln (63) gekoppelt wird,
- von Erfassungsmitteln (63-69; 68, 72, 73, 75, 76) einer Drehmomentermittlungseinrichtung ein Signal erzeugt wird, das dem tatsächlich von dem Behandlungsinstrument und dem Antriebsmotor auf die Mitnahmemittel (63) ausgeübten Drehmoment entspricht,
- dieses Signal bzw. die entsprechenden Drehmomentdaten an Auswertungsmittel eines Steuergeräts geliefert wird bzw. werden,
- das Steuergerät (21) abhängig von diesem Signal die Drehmomentkalibrierung, d.h. Drehmomentkorrektur durchführt und
- das Steuergerät (21) nach dem Drehmomentkalibrierungsvorgang den Antriebsmotor (23) mit einer entsprechenden Leistungszufuhr betreibt.

32. Verfahren nach Anspruch 31,
**dadurch gekennzeichnet,**
**daß** die Mitnahmemittel durch eine mit dem Behandlungsinstrument (38) koppelbare Aufnahmewelle (63) zur mechanischen Übertragung und Erfassung der Drehung eines rotierenden Antriebs des Behandlungsinstruments (38) gebildet sind.

## Claims

1. Medical treatment device (20)
having a control apparatus (21)
a drive motor (23), connectable to the control apparatus (21), with which a treatment instrument (38) can be driven, and
having a torque detection device which, before putting into operation of the drive motor (23) and the treatment instrument (38) coupled therewith, detects the actually exercised torque, or the corresponding torque characteristic line, of the drive motor (23) and the treatment instrument (38),
**characterised in that**,
the torque detection device has
- entraining means (63) for coupling with the treatment instrument (38),
- detection means (63-69; 68, 72, 73, 75, 76) which generate a signal corresponding to the torque actually exercised by the drive motor (23) and the treatment instrument (38) on the entraining means (63), and
- evaluation means (70) which deliver this signal, or the corresponding torque data, to the control apparatus (21),
**in that** in dependence upon this signal the control apparatus (21) carries out the torque calibration, i.e. torque correction, and
**in that** the control apparatus (21), after the torque calibration procedure, operates the drive motor (23) with a corresponding delivery of power.

2. Medical treatment device according to claim 1,
**characterised in that**,
the entraining means are formed by a receiving shaft (63), which can be coupled with the treatment instrument (38), for the mechanical transmission and detection of the rotation of a rotating drive of the treatment instrument (38).

3. Device according to claim 2,
**characterised in that**,
the receiving shaft (63) which can be coupled with the treatment instrument drives a hysteresis brake.

4. Medical treatment device according to any of claims 1 to 3,
**characterised in that**,
the torque detection device obtains the torque data by correlation of the torque of the treatment instrument (38) determined thereby with the power delivered to the drive motor (23).

5. Medical treatment device according to any of claims 1 to 4,
**characterised in that**,
the entraining means (63) translate the rotary movement of the treatment instrument (68) into a displacement force for a displacement medium (67; 72) and
**in that** the detection means further have conversion means (68, 69; 68) which detect a displacement path of the displacement medium (67: 72) brought about by the displacement force and in dependence thereupon deliver the electrical signal.

6. Medical treatment device according to claim 5,
**characterised in that**,
the displacement medium (67; 72) is biased spring elastically.

7. Medical treatment instrument according to claim 5 or 6,
**characterised in that**,
the conversion means (68, 69; 68) detect the displacement path of the displacement medium (67; 72) by means of a switch arrangement which in dependence upon the displacement path of the displacement medium (67; 72) delivers different electrical signals.

8. Medical treatment device according to claim 7,
**characterised in that**,
the switch arrangement (68, 69) includes a plurality of switches which in dependence upon displacement path of the displacement medium (67; 72) are differently actuated.

9. Medical treatment device according to claim 8,
**characterised in that**,
the switch arrangement includes a plurality of resistances (69) combined with the switches (68), so that the switch arrangement has a total resistance value which differs in dependence upon the actuation of the individual switches in dependence upon the displacement path of the displacement medium (67; 72).

10. Medical treatment instrument according to claim 5 or 6,
**characterised in that**,
the conversion means (68, 69; 68) have a potentiometer the resistance value of which changes in dependence upon the displacement path of the displacement medium (67; 72) and which delivers the electrical signal.

11. Medical treatment instrument according to claim 2 and any of claims 5 to 10,
**characterised in that**,
the displacement medium (67; 72) is mechanically coupled with the receiving shaft (63).

12. Medical treatment device according to claim 11,
**characterised in that**,
the displacement means is a leaf spring (67) held at a first end, the second end of which is mechanically coupled with the receiving shaft (63), the conversion means (68, 69) detecting the displacement path of the second end of the leaf spring (67).

13. Medical treatment device according to claim 12,
**characterised in that**,
the second end of the leaf spring (67) is coupled with the receiving shaft (63) via a pre-tensioned cable (66).

14. Medical treatment device according to claim 13,
**characterised in that**,
in the cable (66) there is emplaced a spring elastic means (65) which is stretched in the pulling direction of the cable (66).

15. Medical treatment device according to claim 13 or 14,
**characterised in that**,
the receiving shaft (63) is mechanically coupled with a winding drum (64) to which the cable (66) is attached, so that upon transmission of a rotation of the treatment instrument (38) to the receiving shaft (63) the cable (66) winds up on the winding drum (64).

16. Medical treatment device according to any of claims 12 to 15 and claim 8 or 9,
**characterised in that**,
the plurality of switches (68) are arranged in the longitudinal direction of the leaf spring (67) and thus are differently actuated in dependence upon the deflection of the second end of the leaf spring (67).

17. Medical treatment device according to any of claims 12 to 16 and any of claims 5 to 7,
**characterised in that**,
the switch arrangement (68, 69) can be displaced along the longitudinal direction of the leaf spring (67).

18. Medical treatment device according to claim 11,
**characterised in that**,
the displacement medium is a rotary body (72) mechanically coupled with the receiving shaft (63), the deflection of which rotary body is detected by means of the conversion means (68).

19. Medical treatment device according to claim 18 and claim 8 or 9,
**characterised in that**,
the switches (68) are arranged along the circumferential direction of the rotary body (72) and are actuated by cams (76) which stand out laterally from the outside of the rotary body (72).

20. Medical treatment device according to claim 19,
**characterised in that**,
the switches (68) are arranged uniformly distributed along the circumferential direction of the rotary body (72).

21. Medical treatment device according to any of claims 18 to 20,
**characterised in that**,
elastic return means (73, 79) are provided which in the absence of a torque of the treatment instrument (38) return the receiving shaft and the rotary body (72) into an initial position.

22. Medical treatment device according to claim 21,
**characterised in that**,
the return means include a spiral spring (73) mechanically coupled with the rotary body (72), which spiral spring surrounds the receiving shaft (63).

23. Medical treatment device according to claim 21 or 22,
**characterised in that**,
the return means include a flat spiral spring (79) wound a plurality of times in the rotary body (72), which spiral spring, upon deflection of the rotary body, winds up on the receiving shaft (63) as a result of a torque transmitted to the receiving shaft (63).

24. Medical treatment device according to any preceding claim,
**characterised in that**,
the torque detection device has a housing (71, 77) which has a repository (78) for the treatment instrument (38), whereby a rotating drive of the treatment instrument (38) placed in the repository (78) can at the same time be mechanically coupled with the detection means (63-69; 63, 68, 72, 73, 75, 76) for the detection of the torque exercised by the rotating drive of the treatment instrument (38).

25. Medical treatment device according to claim 24,
**characterised in that**,
the repository (78) has fixing means (80-85) for fixing the treatment instrument (38) in a repository position.

26. Medical treatment device according to claim 25,
**characterised in that**,
the fixing means (80-85) include a recess (85) in which the treatment instrument (38) is to be placed, whereby the recess (85) has a stop edge (84) against which the treatment instrument (38), in the repository position, is pressed and thus fixed by means of an elastically biased element (80), the fixing being releasable by displacement of the elastically biased element (80) against the bias force.

27. Medical treatment device according to any preceding claim,
**characterised by**
display means (75, 76) for the optical display of the torque detected by the torque detection device.

28. Medical treatment device according to claim 27 and any of claims 24 to 26,
**characterised in that**,
the display means have a window (75), formed in the housing (71, 77), through which window the displacement path of the displacement medium (72) can be monitored.

29. Medical treatment device according to any preceding claim,
**characterised in that**,
the torque detection device has a control module for user control.

30. Medical treatment device according to any preceding claim,
**characterised in that**,
the torque detection device is integrated in the control apparatus (21).

31. Method for the operation of a motor-driven medical treatment instrument (38), wherein before the putting into operation of the treatment instrument (38) there is detected, in a torque calibration, the actually exercised torque, or the corresponding torque characteristic line, of the drive motor (23) and the treatment instrument coupled therewith,
**characterised in that**,
- the treatment instrument (38) is coupled with entraining means (63)
- there is generated by detection means (63-69; 68, 72, 73, 75, 76) of a torque detection device a signal which corresponds to the torque actually exercised on the entraining means (63) by the treatment instrument and drive motor,
- the signal or the corresponding torque data is delivered to evaluation means of a control apparatus,
- the control apparatus (1) carries out, in dependence upon the signal, the torque calibration, i.e. torque correction, and
- the control apparatus (21), after the torque calibration procedure, operates the drive motor (23) with a corresponding power delivery.

32. Method according to claim 31,
**characterised in that**,
the entraining means are formed by means of a receiving shaft (63) which can be coupled with the treatment instrument (38) for the mechanical transmission and detection of the rotation of a rotating drive of the treatment instrument (38).

## Revendications

1. Dispositif de traitement médical (20)
avec un appareil de commande (21)
avec un moteur d'entraînement (23) pouvant être fixé sur l'appareil de commande (21), avec lequel on met en fonctionnement un instrument de traitement, et
avec un dispositif de détection du couple, qui saisit avant la mise en service du moteur d'entraînement (23) ainsi que de l'instrument de traitement (38) couplé, le couple réel exercé ou la ligne caractéristique du couple par le moteur d'entraînement (23) et l'instrument de traitement (38), **caractérisé en ce que**
le dispositif de détection du couple présente
- un moyen d'accrochage (63) pour le couplage à l'instrument de traitement (38) ;
- un moyen de détermination (63-69 ; 68, 72, 73, 75, 76), qui produit un signal, qui correspond au moment réel exercé par le moteur d'entraînement (23) et l'instrument de traitement (38), sur le moyen d'accrochage (63), ainsi que
- un moyen d'évaluation (70), qui produit ce signal ou les données correspondantes de couple sur l'appareil de commande (21),
**en ce que** l'appareil de commande (21) réalise le calibrage du couple en fonction de ce signal, à savoir la correction du couple, et **en ce que** l'appareil de commande (21) met en fonctionnement le moteur d'entraînement (23) avec une puissance appropriée après le processus de calibrage du couple.

2. Dispositif de traitement médical selon la revendication 1, **caractérisé en ce que** le moyen d'accrochage est formé d'un axe d'accrochage (63) pouvant être couplé à l'instrument de traitement (38) pour la transmission mécanique et l'acquisition de la rotation d'un entraînement rotatif de l'instrument de traitement (38).

3. Dispositif selon la revendication 2, **caractérisé en ce que** l'axe d'accrochage (63) pouvant être couplé à l'instrument de traitement entraîne une frein à hystérésis.

4. Dispositif de traitement médical selon l'une quelconque des revendications 1 à 3, **caractérisé en ce que** le dispositif de détection du couple obtient les données de couple par corrélation du couple de l'instrument de traitement (38), déterminé par celui-ci, avec la puissance induite par le moteur d'entraînement (23).

5. Dispositif de traitement médical selon l'une quelconque des revendications 1 à 4, **caractérisé en ce que** le moyen d'accrochage (63) transforme le mouvement de rotation de l'instrument de traitement (38) en une force de déplacement pour un moyen de déplacement (67 ; 72) et que le moyen de détermination présente en outre, un moyen de transformation (68, 69 ; 68) qui saisit un déplacement du moyen de déplacement (67 ; 72), engendrée par la force de déplacement et produit le signal électrique en fonction de ce déplacement.

6. Dispositif de traitement médical selon la revendication 5, **caractérisé en ce que** le moyen de déplacement (67 ; 72) est prétendu de manière élastique.

7. Dispositif de traitement médical selon la revendication 5 ou 6, **caractérisé en ce que** le moyen de transformation (68, 69 ; 68) saisit le déplacement du moyen de déplacement (67 ; 72) par un arrangement commutateur, qui donne différents signaux électriques en fonction du déplacement du moyen de déplacement (67 ; 72).

8. Dispositif de traitement médical selon la revendication 7, **caractérisé en ce que** l'arrangement commutateur (68, 69) présente plusieurs commutateurs, qui se comportent différemment en fonction du déplacement du moyen de déplacement (67 ; 72).

9. Dispositif de traitement médical selon la revendication 8, **caractérisé en ce que** l'arrangement commutateur présente plusieurs résistances (69) combinées aux commutateurs (68), de sorte que l'arrangement commutateur présente une valeur de résistance totale différente en fonction du déplacement du moyen de déplacement (67 ; 72), en fonction de l'activation de chaque commutateur.

10. Dispositif de traitement médical selon la revendication 5 ou 6, **caractérisé en ce que** le moyen de transformation (68, 69 ; 68) présente un potentiomètre, dont la valeur de résistance se modifie en fonction du déplacement du moyen de déplacement (67 ; 72), et qui produit le signal électrique.

11. Dispositif de traitement médical selon la revendication 2 et l'une quelconque des revendications 5 à 10, **caractérisé en ce que** le moyen de déplacement (67 ; 72) est couplé mécaniquement à l'axe d'accrochage (63).

12. Dispositif de traitement médical selon la revendication 11, **caractérisé en ce que** le moyen de déplacement est un ressort à lame (67) maintenu à une extrémité, dont la deuxième extrémité est couplée mécaniquement à l'axe d'accrochage (63), où le moyen de transformation (68, 69) saisit le déplacement de la deuxième extrémité du ressort à lame (67).

13. Dispositif de traitement médical selon la revendication 12, **caractérisé en ce que** la deuxième extrémité du ressort à lame (67) est couplée à l'axe d'accrochage (63) par un filin (66) prétendu.

14. Dispositif de traitement médical selon la revendication 13, **caractérisé en ce que** le filin (66) est mis en oeuvre par un moyen élastique (65), qui est tendu suivant la direction de traction du filin (66).

15. Dispositif de traitement médical selon la revendication 13 ou 14, **caractérisé en ce que** l'axe d'accrochage (63) est couplé mécaniquement à un cylindre enrouleur (64), sur lequel est fixé le filin (66), de sorte que le filin (66) s'enroule sur le cylindre enrouleur (64) par transfert d'une rotation de l'instrument de traitement (38) sur l'axe d'accrochage (63).

16. Dispositif de traitement médical selon les revendications 12 à 15 et la revendication 8 ou 9, **caractérisé en ce que** les commutateurs (68) sont disposés en direction longitudinale par rapport au ressort à lame (67) et ainsi, se comportent différemment en fonction de la déviation de la deuxième extrémité du ressort à lame (67).

17. Dispositif de traitement médical selon l'une quelconque des revendications 12 à 16 et l'une des revendications 5 à 7, **caractérisé en ce que** l'arrangement commutateur (68, 69) peut être déplacé le long de la direction longitudinale du ressort à lame (67).

18. Dispositif de traitement médical selon la revendication 11, **caractérisé en ce que** le moyen de déplacement est un corps tournant (72) couplé mécaniquement à l'axe d'accrochage (63), dont la déviation est saisie par le moyen de transformation (68).

19. Dispositif de traitement médical selon la revendication 18 et la revendication 8, **caractérisé en ce que** les commutateurs (68) sont disposés le long de la direction de la périphérie du corps tournant (72) et comportent des saillies (76), qui s'étendent latéralement sur la face externe du corps de rotation (72).

20. Dispositif de traitement médical selon la revendication 19, **caractérisé en ce que** les commutateurs (68) sont répartis régulièrement dans la direction de la périphérie du corps tournant (72).

21. Dispositif de traitement médical selon l'une quelconque des revendications 18-20, **caractérisé en ce que** des moyens de rappel (73, 79) sont prévus, qui en l'absence d'un couple de l'instrument de traitement (38), remettent en position initiale, l'axe d'accrochage et le corps tournant (72).

22. Dispositif de traitement médical selon la revendication 21, **caractérisé en ce que** les moyens de rappel comprennent un ressort à spirale (73) couplé mécaniquement au corps tournant (72), qui entoure l'axe d'accrochage (63).

23. Dispositif de traitement médical selon la revendication 21 ou 22, **caractérisé en ce que** les moyens de rappel comprennent un ressort à spirale plate (79) enroulé plusieurs fois dans le corps tournant (72), qui se déroule sur l'axe d'accrochage (63) lors d'une déviation du corps tournant (72) suite à un couple transmis à l'axe d'accrochage (63).

24. Dispositif de traitement médical selon l'une quelconque des revendications précédentes, **caractérisé en ce que** le dispositif de détermination de couple présente un boîtier (71, 77), qui présente un récepteur (78) pour l'instrument de traitement (38), où un entraînement rotatif de l'instrument de traitement (38) disposé dans le récepteur (78) peut être couplé mécaniquement en même temps au moyen de détermination (63-69 ; 63, 68, 72, 73, 75, 76), pour déterminer le couple exercé par l'entraînement rotatif de l'instrument de traitement (38).

25. Dispositif de traitement médical selon la revendication 24, **caractérisé en ce que** le récepteur (78) présente des moyens de fixation (80-85) pour la fixation de l'instrument de traitement (38) en une position de réception.

26. Dispositif de traitement médical selon la revendication 25, **caractérisé en ce que** les moyen de fixation (80-85) comprennent un creux (85), dans lequel l'instrument de traitement (38) est introduit, où le creux (85) présente un bord de butée (84), contre lequel l'instrument de traitement (38) est pressé et ainsi fixé en la position de réception, par un élément élastique prétendu (80), où la fixation peut être défaite par déplacement de l'élément élastique prétendu (80) à l'opposé de la force de prétension.

27. Dispositif de traitement médical selon l'une quelconque des revendications précédentes, **caractérisé par** des moyens d'affichage (75, 76) pour l'affichage optique du couple déterminé par le dispositif de détermination du couple.

28. Dispositif de traitement médical selon la revendication 27 et l'une quelconque des revendications 24 à 26, **caractérisé en ce que** les moyens d'affichage présentent une fenêtre (75) formée dans le boîtier (71, 77), par laquelle le déplacement du moyen de déplacement (72) peut être contrôlé.

29. Dispositif de traitement médical selon l'une quelconque des revendications précédentes, **caractérisé en ce que** le dispositif de détermination du couple présente un module de commande pour le guidage par l'utilisateur.

30. Dispositif de traitement médical selon l'une quelconque des revendications précédentes, **caractérisé en ce que** le dispositif de détermination du couple est intégré dans l'appareil de commande (21).

31. Procédé de fonctionnement d'un instrument de traitement médical (38) motorisé, où avant la mise en service de l'instrument de traitement (38) lors d'un calibrage du couple, le couple réel et respectivement, la ligne caractéristique du couple, exercé par le moteur d'entraînement (23) et l'instrument de traitement y couplé est déterminé, **caractérisé en ce que**
- l'instrument de traitement (38) est couplé à un moyen d'accrochage (63);
- un signal est produit par un moyen de détermination (63-69 ; 68, 72, 73, 75, 76), d'un dispositif de détermination du couple, lequel signal correspond au moment réel exercé par l'instrument de traitement et le moteur d'entraînement sur le moyen d'accrochage (63),
- ce signal et respectivement, les données correspondantes de couple sont fournies à un moyen d'évaluation d'un appareil de commande,
- l'appareil de commande (21) réalise le calibrage du couple en fonction de ce signal, à savoir la correction du couple, et
- l'appareil de commande (21) met en fonctionnement le moteur d'entraînement (23) avec une puissance appropriée après le processus de calibrage du couple.

32. Procédé selon la revendication 31, **caractérisé en ce que** le moyen d'accrochage (63) est formé par un axe d'accrochage (63), pouvant être couplé à un instrument de traitement (38), pour la transmission mécanique et la détermination du couple d'un entraînement rotatif de l'instrument de traitement (38).
